(19) Europäisches Patentamt / European Patent Office / Office européen des brevets

(11) **EP 1 870 028 A1**

(12) **EUROPEAN PATENT APPLICATION**

(43) Date of publication:
26.12.2007 Bulletin 2007/52

(51) Int Cl.:
*A61B 5/00* (2006.01)     *G01N 21/47* (2006.01)
*G01B 9/02* (2006.01)

(21) Application number: 06461008.2

(22) Date of filing: 23.06.2006

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **OPTOPOL Technology Spolka z o.o.**
**42-400 Zawiercie (PL)**

(72) Inventors:
• **Bajraszewski, Tomasz**
**87-100, Torun (PL)**

• **Wojtkowski, Maciej**
**87-851, Boniewo (PL)**
• **Szkulmowska, Anna**
**85-796, Bydgoszcz (PL)**
• **Targowski, Piotr**
**87-100, Torun (PL)**
• **Kowalczyk, Andrzej**
**87-100, Torun (PL)**

(74) Representative: **Hudy, Ludwik**
**Kancelaria Patentowa Patelha**
**Czernichow 4**
**32-070 Czernichow, Krakow (PL)**

(54) **Apparatus and method for frequency domain optical coherence tomography**

(57)     An apparatus for frequency domain optical coherence tomography comprises an optical frequency comb generator (110) for generating a light beam (210) consisting of discrete spectral components and a spectral analyser (170) which disperses the interference light beam (250) on a multi-element detection device (174) such that each detection element (173) registers at most one of the discrete spectral components.

Fig.8A

EP 1 870 028 A1

## Description

DESCRIPTION

The invention relates to an apparatus for optical frequency domain tomography, especially a device for non-invasive imaging of in vivo anatomical objects, as well as a method for imaging of objects by applying optical frequency domain tomography.

Optical tomography is an imaging technique that can measure differences between a light reference beam and a beam reflected back from an object. Devices and/or methods to produce imaging of objects by means of optical tomography are known in numerous variants. Most of them are described in the references cited below.

1. D. Huang, E. A. Swanson, C. P. Lin, J. S. Schuman, W. G. Stinson, W. Chang, M. R. Hee, T. Flotte, K. Gregory, C. A. Puliafito and J. G. Fujimoto, "Optical Coherence Tomography," Science 254, 1178-1181 (1991)

2. E. A. Swanson, J. A. Izatt, M. R. Hee, D. Huang, C. P. Lin, J. S. Schuman, C. A. Puliafito, J. G. Fujimoto," In vivo retinal imaging by optical coherence tomography," Opt. Lett. 18 (21), 1864-1866 (1993)

3. C. A. Puliafito, M. R. Hee, C. P. Lin, E. Reichel, J. S. Schuman, J. S. Duker, J. A. Izatt, E. A. Swanson, J. G. Fujimoto," Imaging of macular diseases by optical coherence tomography," Ophthalmol. 102 (2), 217-229 (1995).

4. A. F. Fercher, C. K. Hitzenberger, G.Kamp, S.Y.El-Zaiat "Measurements of intraocular distances by backscattering spectral interferometry," Opt. Commun. 117, 43-48 (1995).

5. G. Hausler and M. W. Lindner "Coherence Radar and Spectral Radar - new tools for dermatological diagnosis," J. Biomed. Opt. 3(1),21-31 (1998).

6. R. A. Leitgeb, C. K. Hitzenberger, and A. F. Fercher, "Performance of Fourier domain vs. time domain optical coherence tomography," Optics Express 11, 889-894 (2003).

7. J. F. De Boer, B. Cense, B. H. Park, M. C. Pierce, G. J. Tearney, and B. E. Bouma, "Improved signal-to-noise ratio in spectral-domain compared with time-domain optical coherence tomography," Opt. Lett. 28, 2067-2069 (2003).

8. M. A. Choma, M. V. Sarunic, C. Yang, and J. A. Izatt, "Sensitivity advantage of swept source and Fourier domain optical coherence tomography,"Optics Express 11, 2183 (2003)

9. N. Nassif, B. Cense, B. H. Park, S. H. Yun, T. C. Chen, B. E. Bouma, G. J. Tearney, and J. F. de Boer, "In vivo human retinal imaging by ultrahigh-speed spectral domain optical coherence tomography," Opt. Lett. 29, 480- 482 (2004).

10. S. H. Yun, G. J. Tearney, B. E. Bouma, B. H. Park, and J. F. de Boer," High-speed spectral-domain optical coherence tomography at 1.3 μm wavelength," Optics Express 11, 3598-3604 (2003).

11. N. A. Nassif, B. Cense, B. H. Park, M. C. Pierce, S. H. Yun, B. E. Bouma, G. J. Tearney, T. C. Chen, and J. F. de Boer, "In vivo high-resolution video-rate spectral-domain optical coherence tomography of the human retina and optic nerve," Optics Express 12, 367-376 (2004)

12. R. Leitgeb, L. Schmetterer, F. Berisha, C. K. Hitzenberger, M. Wojtkowski, T. Bajraszewski, and A. F. Fercher, "Real- time measurements of in-vitro flow by Fourier domain optical coherence tomography," Opt. Lett. 29, 171-174 (2004)

13. M.Wojtkowski, A. Kowalczyk, R. Leitgeb, and A. F.Fercher, "Full range complex spectral optical coherence tomography," Opt. Lett. 27, 1415-1418 (2002)

14. R. A. Leitgeb, C.K. Hitzenberger, T. Bajraszewski, and A. F. Fercher, "Phase shifting method to achieve high speed long depth range imaging using FDOCT," Opt. Lett. 28, 2201-2204 (2003)

15. A.H. Bachmann, R.A. Leitgeb, T. Lasser, Heterodyne Fourier domain optical coherence tomography for full range probing with high axial resolution, Optics Express 14(4) 1487-1496 (2006)

16. P. Targowski, I. Gorczyńska, M. Szkulmowski, M.Wojtkowski, A. Kowalczyk, "Improved complex spectral domain OCT for in vivo eye imaging," Opt. Commun. 249, 357-362 (2005)

17. S. R. Chinn, E. A. Swanson, J. G. Fujimoto, Optical coherence tomography using a frequency-tunable optical source, Opt. Lett. 22(5), 340-342 (1997)

18. M. A. Choma, K. Hsu, J. A. Izatt, Swept source optical coherence tomography using an all-fiber 1300-nm ring laser source, J. Biomed. Opt. 10(4) (2005)

19. A. Nussbaum, R. A. Phillips, Contemporary optics for scientists and engineers, Prentice-Hall, Inc., New Jersey 1976, ISBN 0-13-170-183-5, pp. 165-174

20. International Patent Publication No. WO 2004/043245 A1

21. International Patent Publication No. WO 2006/017837 A2

22. International Patent Publication No. WO 2006/036717 A2,

23. US Patent Application Publication No. US 2001/0045513 A1

24. US Patent Application Publication No. US 2002/6493091 B2

25. US Patent Application Publication No. US 2005/0036150 A1

26. United Kingdom Patent Application No. GB 2416451 A

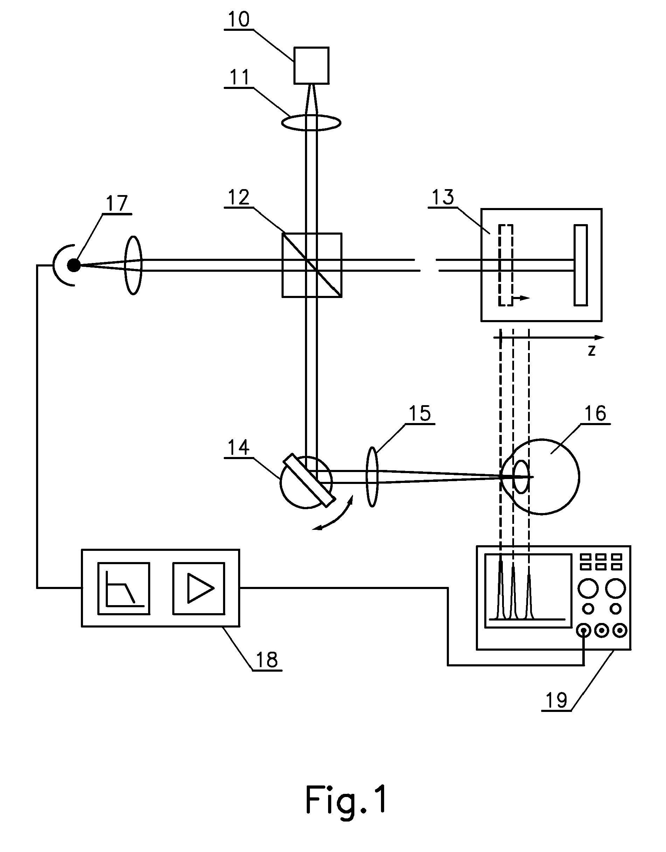

Among the existing solutions there is known a method for optical domain tomography called Time-domain Optical Coherence Tomography (TdOCT) [1-3] applying an arrangement as shown in Fig. 1. This solution belongs to the category of methods of non-invasive and non-contact imaging of the structure of semi-transparent objects and the profile measurement of non-transparent objects. The applied technique is based on analysis of the interference signal of partially coherent light with a wide spectrum using an interferometer, for example, Michelson's interferometer. Light, emitted by a light source 10 and formed by a collimator 11, is split into two arms by means of a beam splitter 12. In an object arm, an object light beam, directed by a traversing scanning mirror 14 and an object lens 15, is directed to and enters an object 16 and, after reflection back from the object's internal structures, returns to the beam splitter 12. Simultaneously, a reference light beam in a reference path is reflected from an adjustable reference mirror 13 and also returns to the beam splitter 12. A resultant light beam is registered by a single photosensitive element 17, which might consist of a photodiode. An electrical signal generated by the photosensitive element 17 is transmitted to an electronic system 18, where it is processed and recorded or displayed on a screen, for example, on an oscilloscope screen 19. The interference signal is observed only if the length of the light-path in each arm is equal. Therefore, in order to receive information about the axial structure of the object along the object beam (conventionally the $z$-axis), it is necessary to change the alignment positions of the movable reference mirror 13 and to register the electrical signal generated by the photosensitive element 17 for each position. An image of a cross-section is obtained by shifting the object light beam perpendicularly to its propagation direction (conventionally the x axis). The range of imaging when applying the TdOCT method is fairly wide and is limited only by the extent of the reference mirror movement. The range of imaging can be selected to suit the application. However, a wide range of imaging necessitates extending the time of signal registration for a given x position of the sampling beam. The speed of examination in optical coherence tomography is defined as the number of measured lines (called A-scans) of the resulting image in one second, which in the TdOCT method is around 800 A-scans/s. This constitutes a serious limitation of this method.

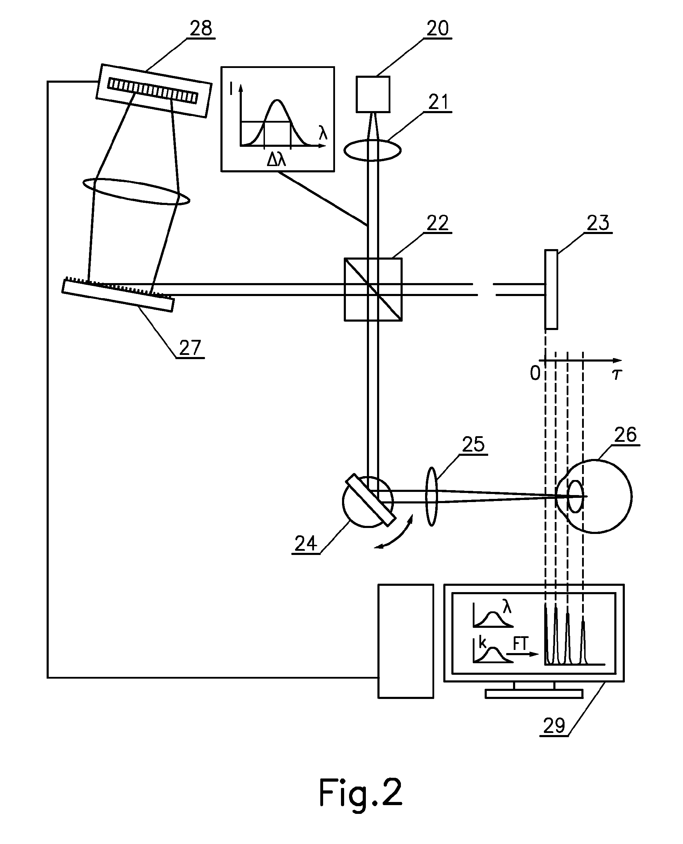

An alternative technique to the above-described method is Spectral Optical Coherence Tomography (SOCT). This method was proposed by Fercher and others [4] and has been described in many other publications [5-16]. Here, instead of single photosensitive element, a spectrometer is used to register a light spectrum, which is modulated by interference fringes. An outline of a scheme using the method of spectral optical tomography is shown in Fig. 2. As in the TdOCT method, light, emitted by a light source 20 and formed by a collimator 21, is split into two arms by means of a beam splitter 22. In the object arm, an object light, directed by a transverse scanning mirror 24 and an object lens 25, penetrates an object 26, is back-scattered by its internal structures and returns to the beam splitter 22. Simultaneously, a reference light beam in the reference arm reflects from a non-moving reference mirror 23 and also returns to the beam splitter 22. The object and reference light beams are combined to form a resultant beam. The resultant beam is next directed to a diffraction grating 27. Then optical components of the resultant beam are separated spatially by the lens and are registered by a matrix 28 consisting of photosensitive elements. The electrical signal generated by the matrix 28 is transmitted to a calculation unit. Information about the axial structure of the object being examined (single image line - A-scan) is received by means of numerical calculation in the calculation unit, for instance by means of a PC 29, first transforming the spectrum from the domain of light wavelength $\lambda$ to the domain of wavenumber $k$ (optical frequencies) on the basis of the Fourier transformation. In SOCT systems CCD linear cameras are most frequently used as spectrum detectors. Thanks to the currently-available high-speed CCD converters, the time for measurement of a single spectrum can be as short as 33 $\mu$s, which results in scanning speed at a rate of 30 000 A-scans/s [15]. Moreover, spectral tomography offers much higher sensitivity due to the fact, that each individual photosensitive cell of the detector registers only a small fragment of the spectrum, which means narrowing the detection band, and thus increasing the signal-to-noise ratio [6-8]. At the same time all wavelengths are registered simultaneously by other photosensitive cells of the detector, therefore exposure time can be effectively extended in comparison with time domain methods, and furthermore additional increase the sensitivity of this method can be achieved. The high speed of the SOCT method allows three dimensional measurements to be made, in which after recording a first cross-section, the beam is traversed in the y axis (perpendicular to the x axis) forming a further trace in direction x. As a final result a three dimensional reconstruction of the object is created.

The basic drawback of the SOCT method is the limited range of imaging, which depends on the wavelength width $d\lambda$ of the spectrum received by each individual pixel of the CCD camera according to the formula:

$$z_{max} = \lambda_0{}^2 / (4\ d\lambda),$$

where $\lambda_0$ is the central wavelength of the light source waveband.

As was stated earlier, in spectral optical tomography analysis of spectral fringes, registered by means of a matrix of photosensitive elements, takes place. The axial range of imaging $z_{max}$ in Fourier domain OCT is associated with the frequency limit of the interference fringes and is defined as that frequency in which two neighbouring interference maxima fall into two neighbouring pixels of the spectrometer detector.

Assuming that the spectrometer detector is able to bridge a range of wavelengths equal to 150 nm, and contains 2048 pixels, then $d\lambda$ will be equal to 150 nm / 2048 = 0.078 nm. Typically $\lambda_0$ is around 830 nm, thus the resulting imaging axial range is 2.2 mm. By increasing the number of pixels in the spectrum detector the axial imaging range may be improved, however, the time needed for a spectrum readout is proportionally extended, which influences the overall measurement time. Additionally there is a loss of usable signals caused by an averaging effect of the interference fringes within the spectral range $d\lambda$ covered by a single photosensitive element.

Another limitation of the SOCT method is the fact, that spectra are registered in the domain of wavelength $\lambda$, whereas a numerical analysis of spectra (Fourier transformation) is made in the domain of wavenumber k. This fact requires calculation of the spectrum from domain A to domain k before making the Fourier analysis. This in turn increases the total time to provide a result, which becomes crucial especially in three dimensional measurements.

Another disadvantage of the SOCT method is the partial loss of the interference signal associated with used detectors. Due to their high operational speed, linear industrial CCD cameras are used in Spectral OCT as spectrum detectors, for example camera type AViiVA M2CL2014 manufactured by Atmel. In this type of camera an effect occurs which is known as leakage, which means that part of the charge collected in a single pixel leaks to neighbouring pixels. This phenomenon causes an additional decrease of spectrometer resolution capacity, therefore it also degrades the useful signal in SOCT.

There are SOCT techniques, so called *Complex Spectral Optical Coherence Tomography* (CSOCT), allowing the range of imaging to be doubled, although at the cost of increasing measurement time. In these methods, from two [15] to five [16] measurements of spectra with shifted phase for a given position x of the sampling beam are made. The main drawback of such methods is their high sensitivity to the instability of spectra phases, thus these solutions are very complicated to apply.

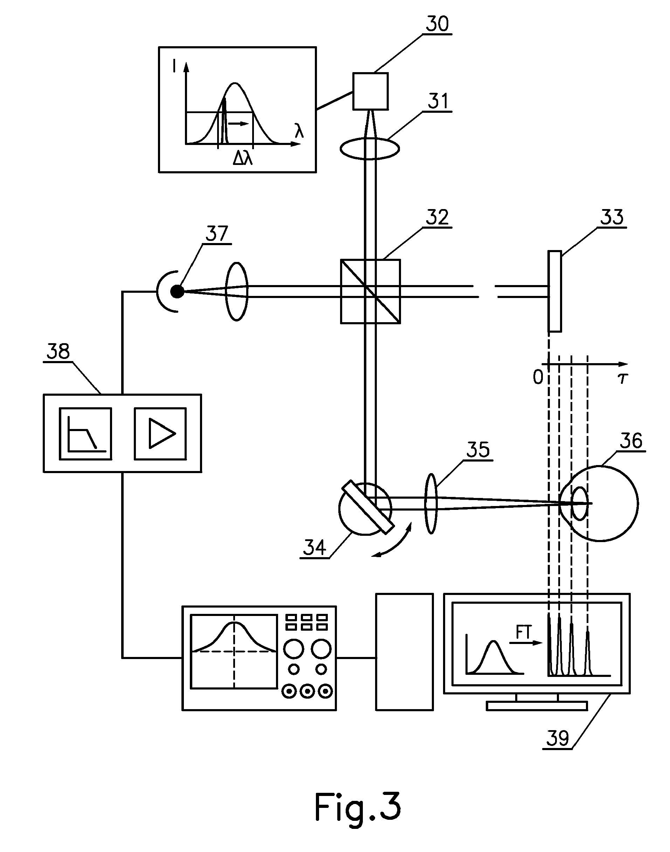

Another version of optical tomography is also known, called *Swept-source Optical Coherence Tomography* (SSOCT) [17, 18], in which a laser light of narrow band, tuned to a wide spectral range, is used, generated by light source 30, as shown in Fig. 3. A light beam is formed by a collimator 31, and is then split into two arms by a beam splitter 32. In the object arm the light, directed by a transverse scanning mirror 34 and an object lens 35, penetrates the interior of an object 36, is back-scattered by its internal structures and returns to the beam splitter 32. Simultaneously, light in the reference arm is reflected from a fixed reference mirror 33 and also returns to the beam splitter 32, in which a resultant beam is created and is registered by a single photosensitive element 37. The electrical signal generated by the photosensitive element 37 is transmitted to an electronic system 38 forming a signal, which may either be recorded by an electronic system 39 or displayed on a screen, for example on the screen of an oscilloscope. In this method, similarly as in SOCT, in order to reproduce information about the axial structure of an examined object, a frequency analysis of the light spectrum emitted by the interferometer is made. However, the method of spectrum detection is different, being made in time by means of a single photosensitive element 37 and simultaneously, the laser light wavelength is scanned. The spectrum is also registered in the domain of wavelength $\lambda$, thus it is necessary to transfer the spectrum to the domain of wavenumber k during digital data processing in the computer.

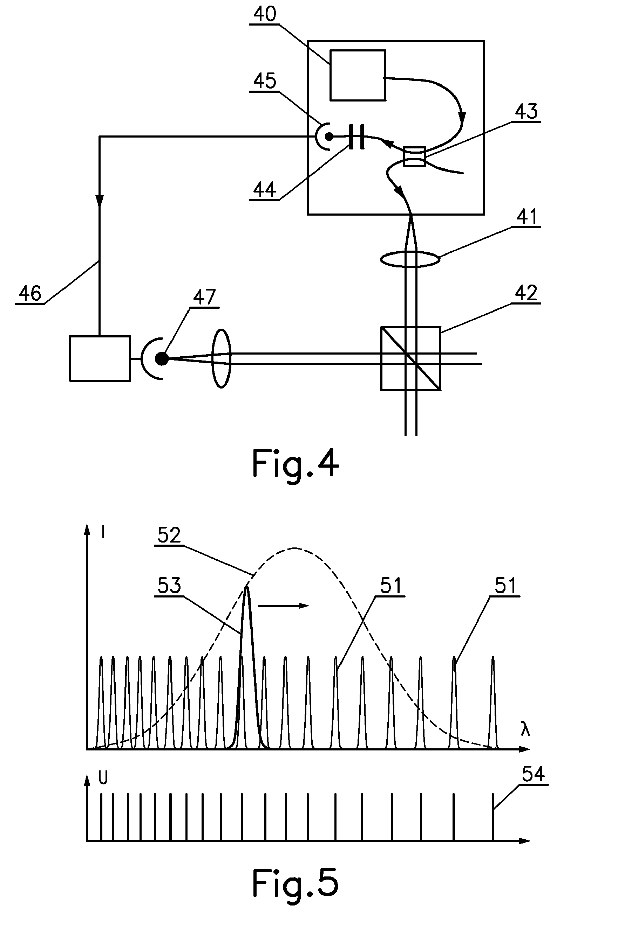

A method SSOCT is also known, in which registration of the spectrum is made directly in the domain of wavenumber k [18]. A device using this method is presented in Fig. 4. Here, a small wave band of light from source 40, by means of a fibre-optical coupler 43, is directed to an etalon 44, for example a Fabry-Perot interferometer with a fixed position between the reflecting planes. The distance between the etalon planes is set in such a way as to create modes 51, densely clustered in range 53 of the light source scanning. Thanks to this, in the moment when equally distanced peaks are generated in the k domain by the etalon 44, a photodiode 45 registers them and converts them into an electric signal $U_{trig}$ 46, consisting of peaks 54. As in other methods, a light beam formed by a collimator 41 is directed to a beam splitter 42, from which beams are directed to an object and a reference mirror. The electric signal $U_{trig}$ 46 triggers the measurement of the intensity of the light returning from the interferometer, which is measured by a single photo-detector 47. The modes 51 and the sequence of electrical signal peaks $U_{trig}$ are shown in Fig. 5. The modes of the etalon are placed in non-equidistant way in domain $\lambda$, but the distances between modes in the k domain are equal, therefore the signals measured by the triggered photo-detector 47 form a spectrum in the domain of wavenumber k. All so far known SSOCT solutions use longer wavelengths, for example 1300 nm, due to technical problems in producing tuned light sources at a wavelength range of 800 nm. This causes a serious limitation to this method, especially when applied for ophthalmic imaging, because in an eye (and especially in measurements near the eye fundus) absorption of light at wavelengths around 1300 nm is much higher than for light at a wavelength range around 800 nm. Moreover, the maximum available longitudinal resolution when using a light source with a wavelength of 1300 nm is as much as 2.5- times smaller than resolution using a 800 nm light source. Another limitation of the SSOCT method is a drop both in the usable signal and the longitudinal resolution caused by the instability of the tuned laser line, known as mode hopping. This also limits the imaging range using this method. Additionally, amplitude modulation of the registered signal occurs, as a result of the instability of the tuned laser system.

An OCT device using optical frequency comb generators [23] is also known. By using two optical frequency comb generators (OFCG) the speed of operation of the TdOCT device is increased by avoiding mechanical scanning. In this system a pair of OFCGs is used, based on modulation of the light beam is so arranged that the signal phase of each signal is different in each arm of the interferometer. Thanks to this, it is possible to generate two signals with optical frequency fringes moved in relation to one another. In such a system, a usable signal carrying information about the structure of an object will be registered only when the optical frequency of the reference arm and the object arm that interfere should ideally overlap one another. By this means it becomes possible to scan in an axial direction without the mechanical element but only by changing the value of the light modulation phase.

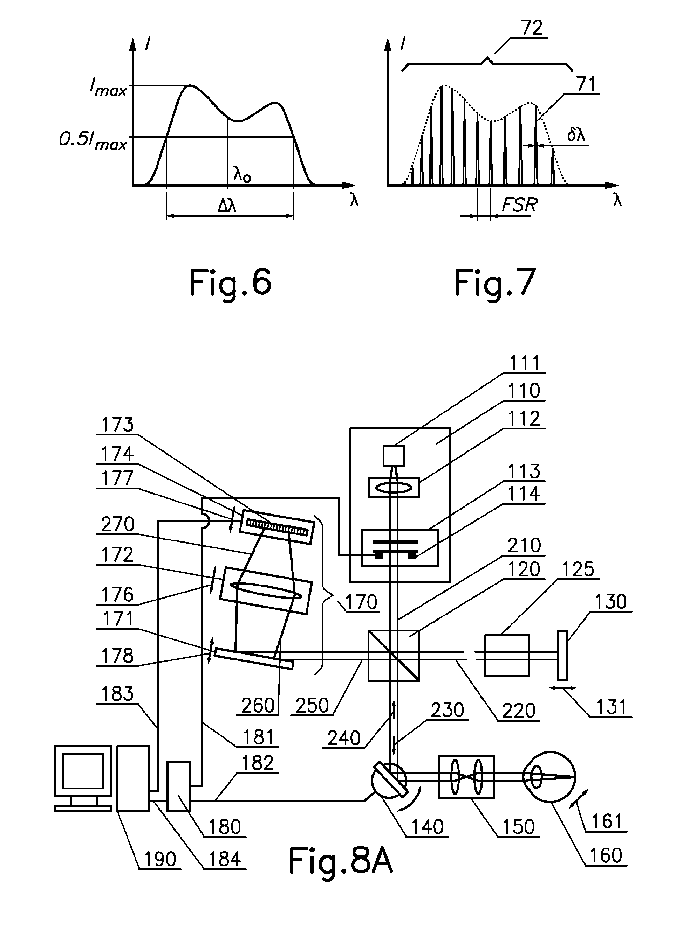

A Fabry-Perot interferometer system [19] is also known. After light with low time coherence passes through the system of tuned Fabry-Perot interferometer, a light spectrum, as shown in Fig. 6, is transformed into an optical frequency comb 72 having components 71 as shown in Fig. 7, which is defined by two parameters:

- Free Spectral Range (FSR) - the spectral distance between the components 71 of the optical frequency comb, which is derived from the formula $FSR = \lambda^2 / (2nd)$. Where d denotes a distance between the reflecting planes of the Fabry-Perot interferometer, and n denotes a refractive index between reflecting planes, which for air is equal to 1;
- $\delta\lambda$ - the spectral band width of a single component 71 expressed by the formula $\delta\lambda = FSR / F$, where $F$ is a parameter called finesse and depends on a reflection coefficient of the reflecting planes of the Fabry-Perot interferometer.

In addition to the imaging techniques described above which form part of these strictly-defined groups, there are many other solutions [20 - 22, 24 - 26] that consist of combinations of parts of the abovementioned methods of object imaging or are modifications of them. For example, a method for fast object-imaging using spectral optical tomography in which, to increase the speed of object imaging, a matrix of sensitive elements with a memory to store information about scanned fragments of the object is described in the publication of international application No. WO 2004/043245 A1.

In turn, the publication of international patent application No. WO 2006/017837 A2 describes a process, system and software arrangement to determine at least one position of one portion of sample using an optical coherence tomography.

Additionally, from the publication of international patent application No. WO 2006/036717 A2 is known an optical feedback between two interferometers for taking desired measurements of length differences of optical paths of two light beams.

Furthermore, the publication of US patent application No. US 2005/036150 A1 describes a method for imaging of concentration and displacement of a specific agent in an examined object using an optical coherence tomography.

In addition, form the publication of UK patent application No. GB 2416451 A is known a method of detecting images of objects using a laser-scanning microscope with linear scanning.

Among the above-described, known imaging techniques, based on the optical tomography method, the best results for eye imaging are achieved by SOCT, characterised by high longitudinal resolution, fast measurement speed and high sensitivity. However, the tomography equipment created to date, based on this method, also has the limitations described earlier, the elimination of which is most desirable.

The idea of the invention is that in an apparatus for optical frequency domain tomography comprising a generating device emitting a light beam with a defined spectrum, a means for splitting the light beam into at least one object light beam and at least one reference light beam, a device forming a combined light beam from at least one reference light beam and at least one object light beam, and a device for spectral analysis of the combined light beam, the generating device emitting the light beam is either a device transforming the light beam with the defined spectrum into a set of components or an optical frequency comb having components spaced at discrete defined distances, or in a path of the light beam emitted by the generating device or in the paths of at least one reference light beam and at least one object light beam is placed a device transforming the light beam or the reference light and object light beams into a set of components or an optical frequency comb or optical frequency combs, as applicable, having components spaced at discrete defined distances, whereas the means for splitting the light beam into at least one object light beam and at least one reference light beam and the device forming the combined light beam from at least one reference light beam and at least one object light beam are capable to process the optical frequency combs, and the device for spectral analysis of the combined light beam is provided with a multi-element detection device having photosensitive elements capable of registering more than one component of the optical frequency comb, and each of the sensitive elements of the multi-element detection device registers at most one component of the optical frequency comb, and wherein the photosensitive elements of the multi-element detection device are spaced at distances correlating with the discrete defined distances between the components of the optical frequency comb.

Preferably, the device transforming the light beam with the defined spectrum into the optical frequency comb or the set of the optical components is a Fabry-Perot interferometer or an adjustable or tuned scanning Fabry-Perot interferometer or an etalon that comprises an apparatus for changing a distance between reflective mirror planes.

Preferably, the means for splitting the light beam and/or the device forming the combined light beam from at least one reference light beam and at least one object light beam is either at least one cube or rectangular prism beamsplitter or at least one fiber-coupler or at least one semi-transparent mirror.

Preferably, in the path of the object light beam is placed at least one element focusing the object light beam.

Preferably, in the path of the object light beam and/or the reference light beam is placed at least one optical arrangement modifying a cross-section shape of the object light beam and/or the reference light beam.

Preferably, in the path of the object light beam is placed an arrangement adjusting the position of the object light beam relative to the examined object.

Preferably, an object is placed on an apparatus adjusting a position of the object relative to the object light beam.

Preferably, in the path of the reference light beam is placed a reference mirror having an adjustable position.

Preferably, in the path of the reference and/or the object light beam is placed an arrangement to equal the path-lengths of the object light beam and of the reference light beam.

Preferably, the device for spectral analysis of the combined light beam comprises a means for separating spatially the combined light beam into a resultant spectrum.

Preferably, the multi-element detection device is a matrix or a line of photosensitive elements.

Preferably, the device for spectral analysis of the combined light beam is provided with a calculation unit for transforming the resultant spectrum into an image of the object.

Preferably, the device transforming the light beam with a defined spectrum into the optical frequency comb or the set of the optical components having the components spaced at discrete, defined distances is fitted with a positioning device adjusting the distances between the optical components of the set of the optical components to the distances between the photosensitive elements of the multi-element detection device.

Preferably, the device for spectral analysis of the combined light beam is fitted with an arrangement for adjusting the distances between the optical components of the set of the optical components to the distances between the photosensitive elements of the multi-element detection device.

The idea of the invention is also a method for imaging objects by applying optical frequency domain tomography, using the invented apparatus, while the elements of the invented apparatus mentioned here (such as the device converting the light beam or the reference light and object light beams into the optical frequency comb or the set of optical components) may be attained using hardware or software, separately or in combination. The proposed method comprises generating a light beam with a spectrum consisting of a set of optical components and/or forming an optical frequency comb, splitting the set of optical components into at least one reference set of optical components and at least one object set of optical components, directing at least one object set of optical components onto an object and forming a returning object set of optical components, transforming at least one reference set of optical components and at least one returning object set of optical components into a combined light beam, forming a spectrum of the combined light beam having optical resultant components spaced at discrete, defined distances, registering the spectrum of the combined light beam by a multi-element detection device fitted with photosensitive elements spaced at distances correlated with the discrete, defined distances of the optical resultant components of the combined light beam and transforming the spectrum of the combined light beam into a data stream enabling the visualisation of the object being examined.

Preferably, generating the light beam in the form of the optical frequency comb or the set of the optical components is conducted by a Fabry-Perot interferometer or an adjustable or tuned scanning Fabry-Perot interferometer placed in a generating device emitting the light beam or after the generating device emitting the light beam.

Preferably, generating the light beam as the optical frequency comb or the set of the optical components is achieved by a phase or amplitude modulator placed in a generating device emitting the light beam or after the generating device emitting the light beam.

Preferably, generating the light beam as the optical frequency comb or the set of the optical components is characteristic of the type of light emission for the light source used.

Preferably, forming the spectrum of the combined light beam is done by a spectrometer.

Preferably, registering the spectrum of the combined light beam is performed by a multi-element detection device fitted with photosensitive elements.

Preferably, a distance between reflective planes of the Fabry-Perot interferometer or the adjustable or tuned Fabry-Perot interferometer is changed to position successive components of the resultant spectrum, this being the set or group of component modulated by the interference signal, onto every q-th photosensitive element of the multi-element detection device, where $q > 0$.

Preferably, an angle between the combined light beam and the device separating spatially wavelength components is chosen to fit successive components of the resultant spectrum, this being the set or group of component modulated by the interference signal, onto every q-th photosensitive element of the multi-element detection device, where $q > 0$.

Preferably, an angle of incidence of the resultant spectrum onto the multi-element detection device is chosen to align successive components of the resultant spectrum, this being the set or group of component modulated by the interference signal, onto every q-th photosensitive element of the multi-element detection device, where q > 0, especially if q = 2.

Preferably, from signals generated by components of the resultant spectrum projected onto the multi-element detection device, this being the set or group of component modulated by the interference signal, are chosen signals from every *q-th* photosensitive element of the multi-element detection device.

Preferably, transforming spectra into a visual or virtual image of objects is performed by calculating Fourier transformations and imaging all Fourier transformations as a light intensity image.

The proposed invention for the device for spectral optical tomography in optical frequency domain and the method of objects imaging by means of using an instrument for spectral optical tomography in optical frequency domain eliminates earlier described disadvantages of device for spectral optical tomography SOCT, which are: limited range of imaging, drop of usable signal in the function of interference fringes frequency, necessity to calculate the spectrum from the domain of wavelength $\lambda$ to the domain of wavenumber k and drop of usable signal caused by leakage effect in used CCD cameras.

The invention will now be described by way of example and with reference to the accompanying drawings in which:

Fig. 1 shows a block diagram of a system adopting a technique of Time-domain Optical Coherence Tomography (TdOCT) known in the prior art;

Fig. 2 shows a block diagram of a system adopting a technique of Spectral Optical Coherence Tomography (SOCT) known in the prior art;

Fig. 3 shows a block diagram of the first embodiment of a system adopting a technique of Swept-source Optical Coherence Tomography (SSOCT) known in the prior art;

Fig. 4 shows a block diagram of the second embodiment of a system adopting a technique SSOCT known in the prior art;

Fig. 5 shows a change of intensity of laser light with respect to wavelength and a change of trigger voltage $U_{trig}$;

Fig. 6 shows a spectrum of a light beam;

Fig. 7 shows an optical frequency comb;

Figs. 8A, 8B, 8C show block diagrams of arrangements for imaging of objects applying spectral optical coherence tomography using an optical frequency comb;

Fig. 9 shows a spectrum of a light beam modulated by an optical frequency comb;

Fig. 10 shows a spectrum of a combined light beam;

Fig. 11 shows dependency of a shifting value $\phi$ on a distance *d* between reflecting planes of an adjustable Fabry-Perot interferometer;

Fig. 12 shows the idea of shifting of components of an optical frequency comb;

Fig. 13 shows a flow chart of a method for imaging of objects using optical frequency domain tomography;

Fig. 14 shows a flow chart of interference;

Fig. 15 shows a flow chart of an algorithm for detecting of a spectrum;

Fig. 16 shows a flow chart of further variant of an algorithm for detecting of a spectrum;

Fig. 17 shows a flow chart of the first variant of data analysis;

Fig. 18 shows a flow chart of further variant of a method for imaging of objects using optical frequency domain tomography;

Fig. 19 shows a flow chart of yet further variant of a method for imaging of objects using optical frequency domain tomography;

Fig. 20 shows a flow chart of another variant of data analysis;

Figs. 21 - 23 show methods of imaging of spectrum onto a matrix of a multi-element detection device;

Fig. 24 shows a method of multiplexing a resultant multiple spectrum;

Fig. 25 shows an electrical signal to control of positions of galvanometric mirrors of an optical system for changing light beam direction, an electrical signal to control an adjustable Fabry-Perot interferometer and an electrical signal to a multi-element detection device;

Fig. 26 shows a way to form a difference spectrum; and

Fig. 27 shows the Fourier transformation of an individual line of an object cross-section.

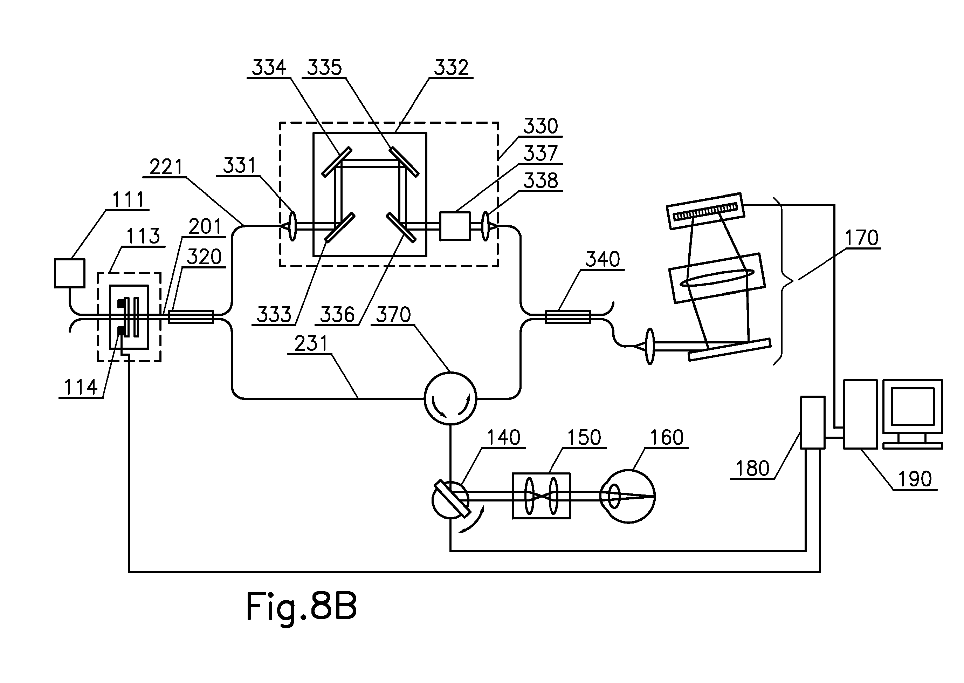

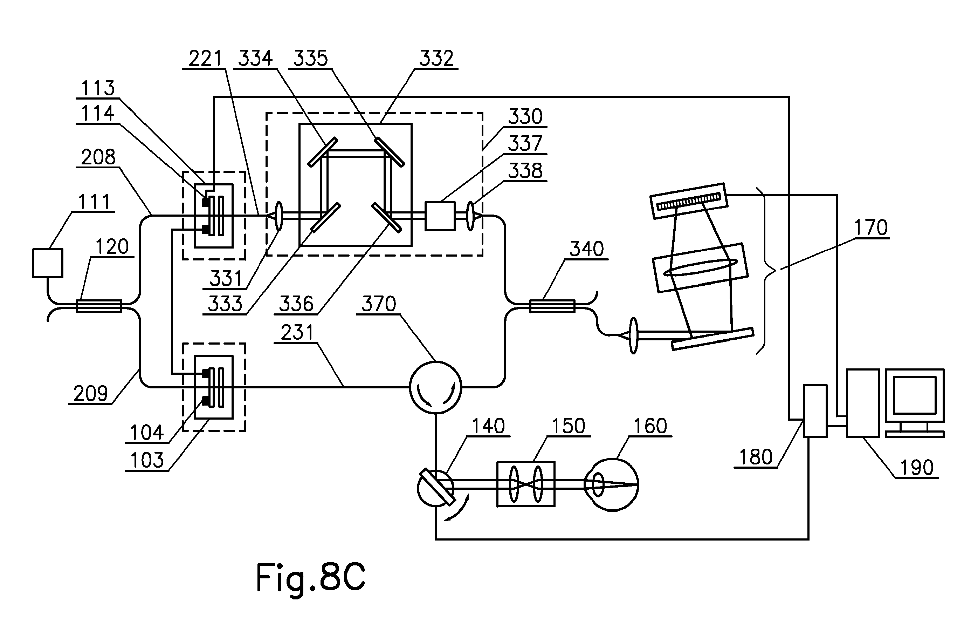

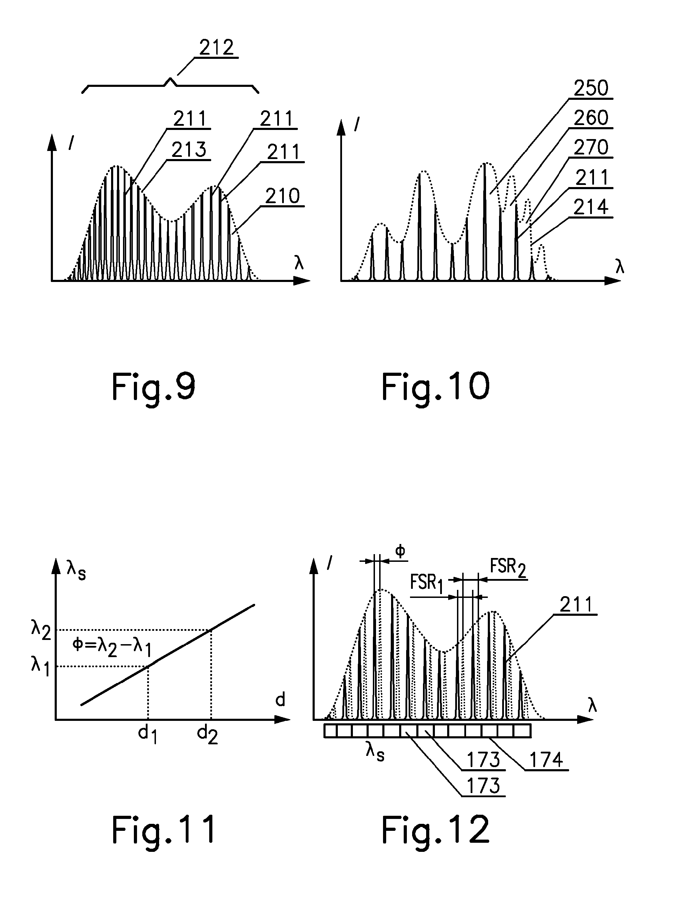

The presented invention, shown in Figs. 8A, 8B, 8C, is a device for imaging objects using Fourier domain optical coherence tomography. This is an apparatus for optical frequency domain tomography comprising, among others, an optical frequency comb generator 110 emitting a light beam 210 consisting of discrete spectral components 211, as shown in Fig. 9, equidistantly distributed in optical frequency domain that form an optical frequency comb 212 or a component set having an envelope 213 of a family of components that is directed through maximum values of light intensity of the discrete spectral components 211 in short components, and is displayed as a light-beam spectrum or as a dependency of the light intensity on wavelength $\lambda$. The generating device or the optical frequency comb generator 110 is provided with a light source 111 emitting a light with low temporal coherence and high spatial coherence with a range or a spectral band $\Delta\lambda$ and a central wave length $\lambda_0$, an optical element 112, such as a collimating lens, and an adjustable or tuned Fabry-Perot interferometer 113, which in specific cases is called an etalon. One of the mirrors of the adjustable Fabry-Perot interferometer 113 is mounted on a positioning device 114 to control positions of the components 211 of the optical frequency comb and/or to adjust distances between the components 211 of the component set to distances between photosensitive elements of a matrix, description of which will follow. Such a positioning device might be a piezo-electric element, which is modified after being affected by applied voltage and altering, at the same time, the distance between the mirrors of the adjustable or tuned Fabry-Perot interferometer 113. If the device is constructed using fibre technology the adjustable Fabry-Perot interferometer is a commercially available device.

The generator 110 of the optical frequency comb or the components set can be made as a light source emitting the optical frequency comb. The generator 110 of the optical frequency comb can also be used with laser light with high temporal coherence with the use of phase or amplitude modulators. Thus, optical components resulting from modulations of the phases of the input light are created.

The light beam 210, generated by the generator 110 of the optical components or the optical frequency comb, passes through a beam splitter 120. The light beam 210 is split by the beam splitter 120 into at least one reference light beam 220 and at least one object light beam 230. The reference light beam 220 passes through a system of optical elements 125 to compensate for dispersion in both arms of the apparatus, and is directed to a reference mirror 130 and is then returned by the reference mirror 130. As it returns, the reference light beam again passes through the system of optical elements 125 for compensation of dispersion and again reaches the beam splitter 120.

The object light beam 230 is directed onto an object by an optical system 140 for changing beam direction, in which at least one mirror is mounted on a pivoted mechanism, and by a set of optical elements 150 that forms the object light beam 230, dependant on the selected/specific application and/or polarizes the object light beam 230. For instance, for examination of the eye fundus a set of two lenses in a telescopic arrangement may be applied. A returning object light beam 240, with a circular, elliptical or linear cross-section, backscattered or reflected from the internal structures of the object 160, is collected by the set of optical elements 150 and is directed to the beam splitter 120 by the optical system 140 for changing beam direction. The object light beam 240 returning from the object and the reference light beam 220 reflected from the reference mirror 130 is assembled in the beam splitter 120 into a combined light beam 250, whose spectrum is shown in Fig. 10. The combined light beam 250 is then analysed by a spectrometer 170 consisting of a diffraction grating 171, a system of optical elements 172 forming an arrangement for adjusting the distances between the components 211 of the component set to the distances between the photosensitive elements 173 of the matrix, which may be a component of a multi-element detection device 174, for example a CCD device. In the spectrometer 170, the combined light beam 250 is transformed into a resultant spectrum 260 by a device separating spatially wavelength components such as the diffraction grating 171. The resultant spectrum passes through the system of optical elements 172 and is then displayed on the matrix of photosensitive elements 173, which transforms the displayed spectrum 270 into electrical signals for further analysis. The spectrometer may have a device 176 to align the optical elements 172 and a device 177 to control a position of the multi-element detection device 174 as well as a device 178 to control a position of the spectrometer 170 relative to the combined light beam 250.

Figs. 8B and 8C show an optic-fibre configuration of the apparatus for optical frequency domain tomography, with the Fabry-Perot interferometer 103, 113 that is aligned with the light beam 201 fitted with a positioning device 104, 114 and placed in front of a fiber-coupler 320 or aligned with the reference light beam 221 and the object light beam 231. In the optic-fibre configuration, the reference light beam 221 passes through an optical device 330 that forms the reference light beam or passes through the group of optical elements which compensate or correct dispersion in both arms of the interferometer and/or passes a light-polarizer, and next, the reference light beam is directed to a fiber-coupler 340. The optical device 330 may contain an optical element 331 such as a collimator, or a delay system, possibly a combination of four mirrors 333 - 336, a group of optical components 337 for compensation of dispersion in both arms of the apparatus and an optical element 338, such as a focussing lens. In turn, the object light beam 231 is directed firstly onto the object to be inspected by a 3-port linear optical circulator 370, the optical system 140 for changing beam direction, and then passes the arrangement of optical elements 150 that conforms the object light beam in accordance with the intended purpose of the investigation, like in the optic-open configuration. The optical system 140 for changing beam direction may also be provided with an adjustable light-filter to restrict the intensity of the object light beam during the detection of the background spectrum.

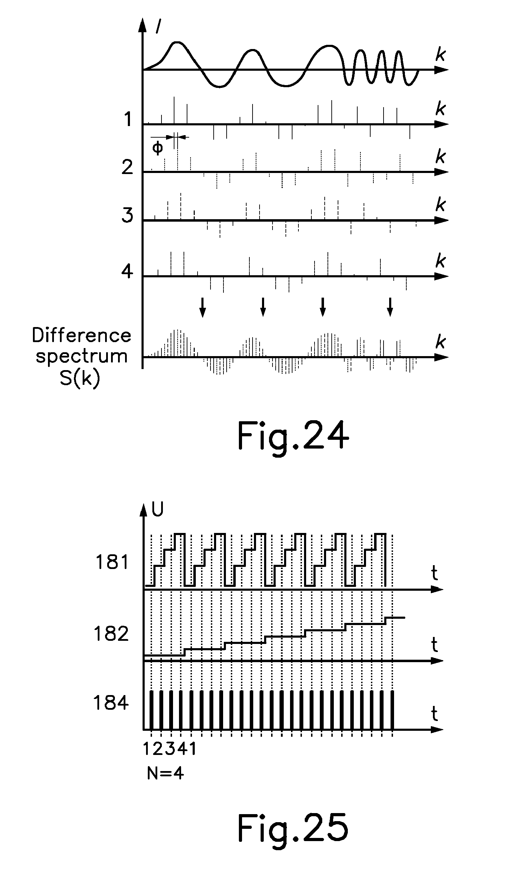

Additionally, the apparatus for optical frequency domain tomography within the spectral frequency band contains a control system 180 generating signals 181, shown in Fig. 25, for the generator of the optical components or the optical frequency comb in order to control the positions of the components 211 within the spectrum of the light beam 210. To control the positions of the optical components 211 in the light beam 210, the control system 180 has a signal generator to produce a signal 181 which controls the device 104,114, for example a piezo-electric element, to vary the distances between the reflective planes of the tuned Fabry-Perot interferometer 103, 113.

Additionally, the control system 180 generates a control signal as shown in Fig. 25 to control the optical system 140 for changing light beam direction. This may consist of a set of galvanometric mirrors, changing the direction of the object light beam 230. The control signal 182 sets the galvanometric mirrors that direct the object light beam 230, containing the optical frequency comb 212, onto the object 160 to be examined in a given position.

The device for spectral optical tomography in the domain of optical frequencies also contains a calculation unit 190, for example a PC, which communicates with the control system 180, collects and stores data registered by the CCD device or the multi-element detection device 174 and contains software for digital data processing, converting the data into a three dimensional image of the object being examined.

Cross-sectional imaging, using the device for optical frequency domain tomography, uses a method of signal measurement by applying the optical frequency comb, which is based on multiple (or in certain circumstances single) registration of the optical frequency comb with simultaneous changes of its components position. For displaying a complete cross-section of the object, the object light beam must be moved along the object in a direction perpendicular to the direction of the object beam propagation and for every position of the object beam a signal must be registered and converted into data, which after processing enables the creation of the complete cross-sectional image.

It is essential that the optical frequency comb, consisting of discrete, equidistant, narrowly separated, spectral components may be set up in two ways, namely, either by using a broadband light source and frequency modes as a discriminatory tool or using optical frequency comb generators based on phase modulation and an additional reflective cavity. In the first arrangement it is possible to separate light of different wave-lengths by using interference phenomena as per the Fabry-Perot experiment. In the second case the generation of the optical frequency comb is based on high-power electro-optic modulators, which can impose dozens of sidebands on a single-frequency input beam from a single-frequency continuous-wave laser. It is also known that this process can be made more efficient by placing the modulator in a resonant cavity, particularly when intracavity dispersion has been reduced to a minimum. Either method of optical frequency comb generation can be used in the described invention.

Also an essential component of this invention is possibility to adjust and control a position of the whole comb in the optical frequency domain. Figs. 11 and 12 show the idea of shifting the entire comb on the $\lambda$ axis by an increment of ø, shown in Fig. 24, and multiplexing the resultant spectrum signals. The optical frequency comb can be moved, for example, by using the tuned Fabry-Perot interferometer, in which the distance between the interferometer mirrors is variable. In this case changes of the distance d between the reflecting planes in the tuned Fabry-Perot interferometer influence the value of ø of the movement of the optical frequency comb and the distance FSR between neighbouring optical components As of the optical frequency comb or the set of components. However the difference between $FSR_1$ for the distance $d_1$ between the reflecting planes of the tuned Fabry-Perot interferometer and $FSR_2$ for distance $d_2$ is of no consequence because it is much lower than the value of the complete shift by the value of ø of the optical frequency comb. The value of the position alteration ø of the optical frequency comb can be random, but must be known, because it is used during the digital data processing.

The steps of the methods described bellow do not necessarily need to be performed serially and may be performed simultaneously.

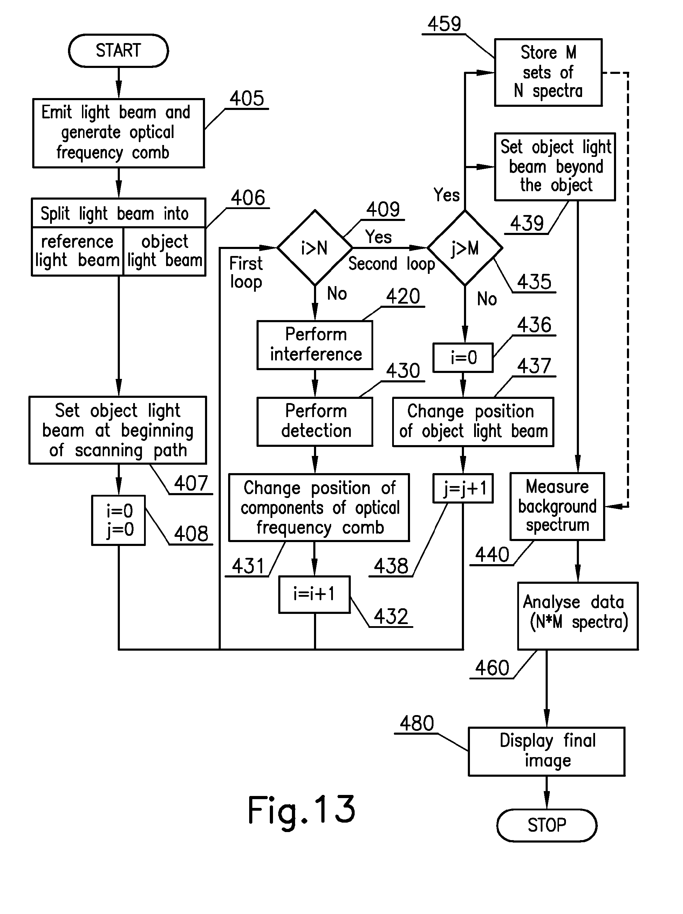

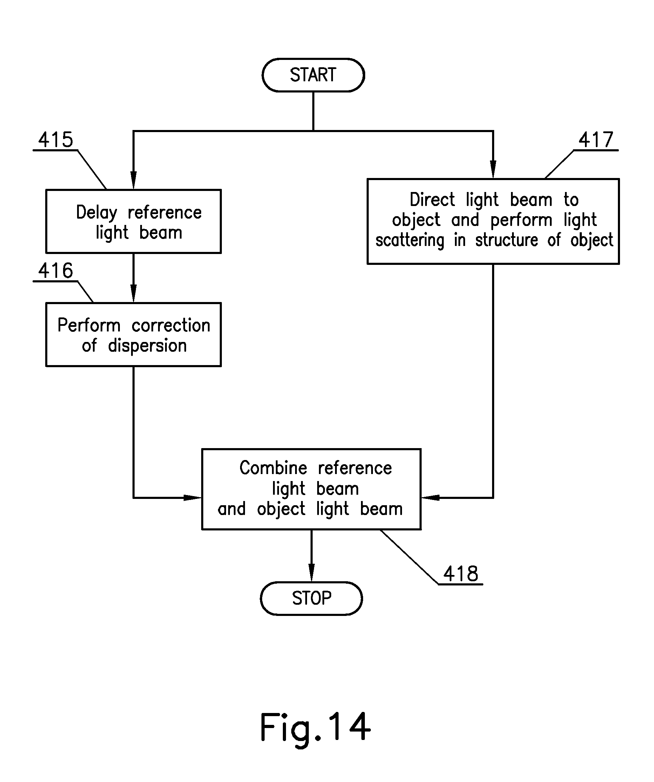

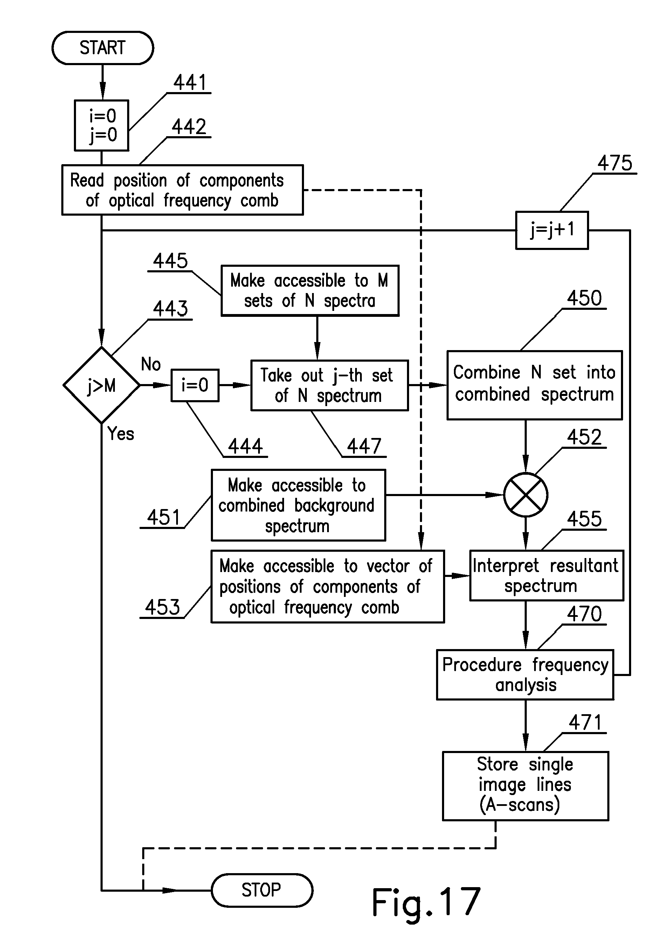

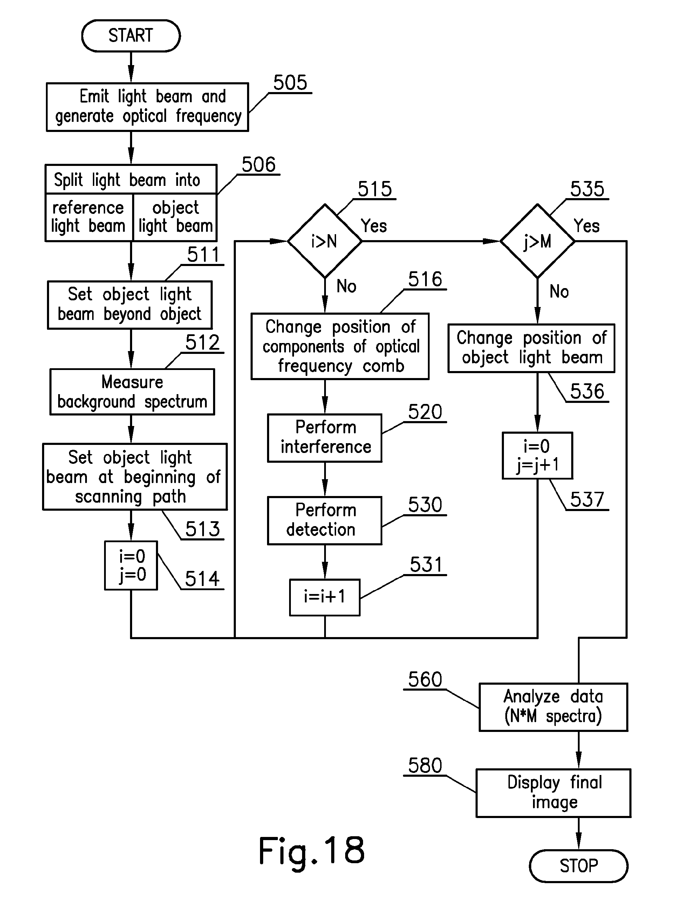

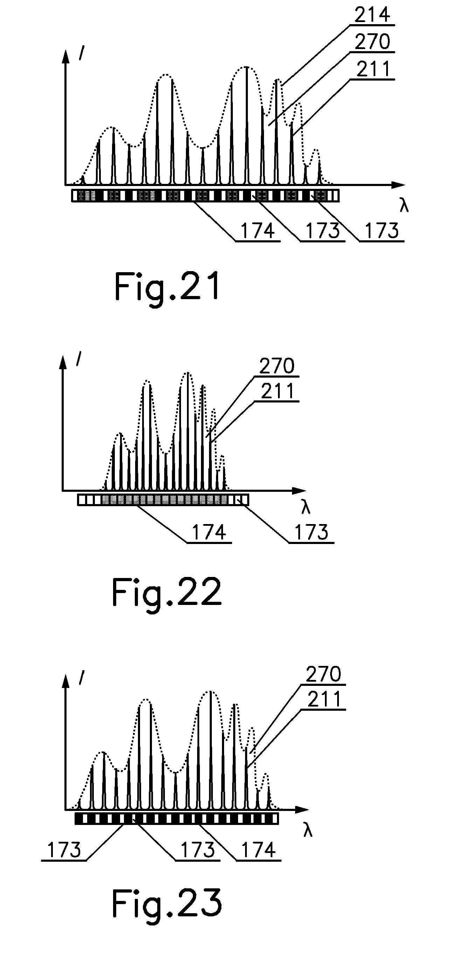

The method for imaging of objects using optical frequency domain tomography is shown in Fig. 13. First, in step 405, the light beam is generated and the optical frequency comb is formed. Then, in step 406 the light beam is split into the reference light beam and the object light beam and in step 407 the object light beam is set to initiate the scanning path. It is possible that the optical frequency comb is formed in the reference light beam and the object light beam after splitting the light beam generated by the light source. In step 408 i and j are set to zero and in step 409 i and N are compared if $i$ is bigger than N. If $i$ is lower than N or equal to N, interference, shown in Fig. 14, consisting of steps 415-418, is performed in step 420. In step 430 the spectrum is identified. The spectrum is detected and registered by the multi-element photo-detector or the multi-element detection device in such a way that one discrete photosensitive element of the multi-element detection device detects and registers not more than one component of the optical frequency comb. The detection and registration of the spectrum is described in detail in the text with reference to Figs. 21 - 23. Next, in step 431 the position of the mirrors of the tuned Fabry-Perot interferometer is set thereby the positions of the components of the optical frequency comb are changed by the predefined and known value ø. In step 432 $i$ is increased by 1. Steps 420 - 431 form the first loop and are repeated N times. The optimum number of repetitions $N$ is equal to the value of ratio $FSR$ to ø, and the method for changing the positions of the optical frequency comb is presented in Fig. 24. Changes of optical frequency comb positions are caused by the electric signal 181, as presented in Fig. 25 synchronized with electric signals 182, 184. If the value of the ratio FSR to ø is not an integer, the shift distance/value ø must be modified in such a way as to make $N$ = FSR / ø a whole number. In step 435 it is checked whether j is higher than M. If $j$ is lower than M or equal to M, i is set to zero in step 436, the position of the object light beam is changed in step 437 and in step 438, j is increased by 1 and the procedure is moved to step 409. Steps 409 - 438 form the second loop and are repeated M times. In this loop a number of iteration M means a number of lines of the resulting cross-sectional image of the object being examined and depends on the purpose of the examination. When the object light beam reaches the end of the scanning path, M sets consisting of N spectra is registered. Then, the procedure is moved to step 439, in which the object light beam is moved beyond the object or is blanked off or is turned-off. In step 440 a background spectrum is determined and data analysis of M sets consisting of N spectra is performed in step 460. The data analysis is shown in details in Fig. 17 and in Fig. 18. Then, the procedure is continued in step 480, in which all calculated lines called A-scans are displayed side by side, a final result being a cross-section or a three dimensional representation of the object, depending whether scanning of the object by the object light beam was in one, or in two axes perpendicularly to the direction of light propagation in the object light beam.

In an embodiment, in which the reference mirror is coupled to the positioning device 131 capable of moving the reference mirror by a distance equal to a light wave length, there is a possibility to perform an additional measurement for each position of the object light beam by changing the position of the reference mirror by a distance equal to a fraction of the optical wave length. This allows several A-scans to be taken at the same position of the object light beam and due to this it is possible to determine a phase as well as an amplitude of the interference signal as in the Complex Spectral OCT method.

Fig. 14 illustrates the interference of at least one object light beam and at least one reference light beam, in the shape of the optical frequency comb with an envelope surrounding the maxima of light intensity at particular component wave lengths, which has a form corresponding to the shape of the spectrum of the light beam generated by the light source. The interference, presented in Fig. 13 as step 420, begins by forming the reference light beam and the object light beam after splitting the light beam generated by the light source. In step 415 one of the light beams, modified to have a shape of the optical frequency comb, is delayed to equalise a travel time of the reference light beam moving along a reference light beam path and a travel time of the object light beam moving along an object light beam path that extends to the first selected layer of the object being examined. Then, in step 416 the dispersion is corrected and in step 417 at least one of the light beams is directed onto the object and focused on it. Next, in step 418, the object light beam, scattered off and reflected from the internal structures of the object, is combined with the reference light beam to form a combined light beam, in which the individual components of the optical frequency comb of the reference light beam interfere with the respective components of the optical frequency comb of the object light beam returning from the object.

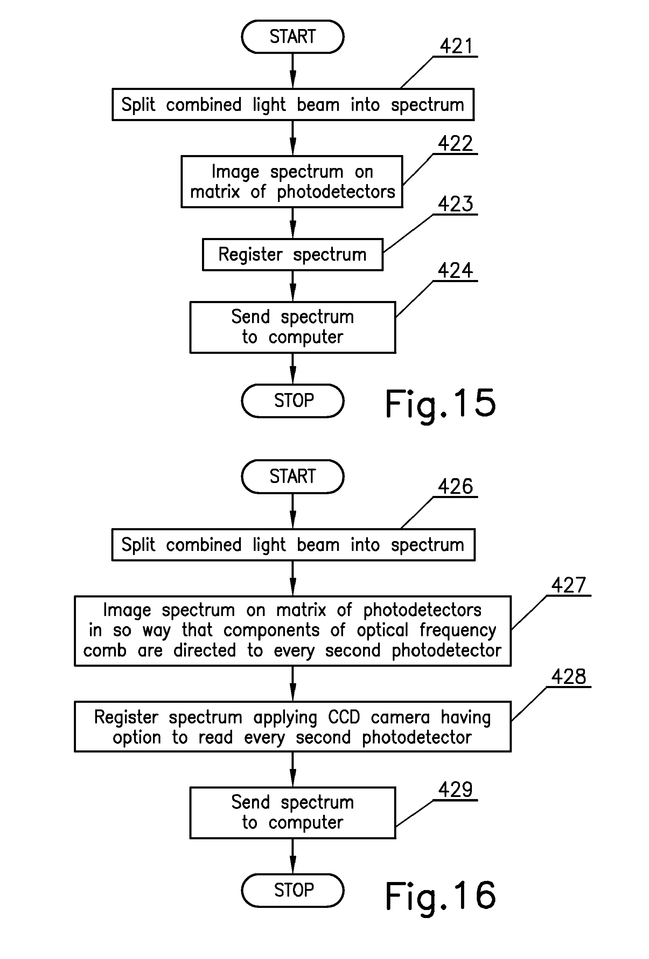

Fig. 15 shows one of embodiments of an algorithm for detecting of the spectrum. First, in step 421 the components of the optical frequency comb of the combined light beam, formed from components of the optical frequency comb of the reference light beam and components of the optical frequency comb of the object light beam, are split spatially to form a resultant spectrum, which is projected onto the multi-element photo-detector or the photosensitive elements of the multi-element detection device in step 422. In step 423 the resultant spectrum is registered by the multi-element photo-detector in such a way that one discrete photosensitive element of the multi-element photo-detector registers not more than one component of the optical frequency comb of the resultant spectrum. In other embodiment, the design of the system is such that one discrete photosensitive element of the multi-element photo-detector detects and registers only a part of one component of the optical frequency comb of the combined light beam and that means that one component of the optical frequency comb of the combined light beam is detected and registered by few discrete photosensitive elements.

An electrical signal is generated in the discrete photosensitive element of the multi-element photo-detector. This signal corresponds to the light intensity resulting from the interference of a particular component of the optical frequency comb of the reference light beam and its corresponding component of the optical frequency comb of the object light beam returning from the object what means that only components of the same ranges of wave length can interfere with each other. At least, in step 424 the electrical signal generated in the multi-element photo-detector is digitised and transferred to the processing arrangement, for example a calculation unit.

It is possible to perform the spectrum detection in several ways. The first possibility, as presented in Fig. 21, is such, that the components of the optical frequency comb fall upon the multi-element photo-detector, for example the matrix 174 of photosensitive elements in a free way, but in such a way, that a single cell or a single pixel or a single photosensitive element registers not more than one component. The advantage of such a configuration is that no digital calculation of spectra between wavelength $\lambda$ and wavenumber $k$ is required. Choice is only made of interpolated points from every registered spectrum in a way described from step 442 to 455 of the algorithm presented in Fig. 17. The process of interpolating the choice of points is about 70% less time-consuming than the currently-applied method for the digital calculation of spectra from $\lambda$ to $k$ by using an analytical formula. Another advantage of this configuration is to minimise the effect of averaging the interference fringes after sectioning the wave-lengths registered by a single pixel of the matrix of photosensitive elements. This is possible because the single pixel of the matrix receives an approximately monochromatic wave, this being an effect of using the optical frequency comb as a light source in a device for spectral optical tomography operated at optical frequencies. In such a configuration it is also possible to make measurements of N spectra that are combined into a single resultant compound spectrum as described. Thanks to this, the range of imaging can be increased N-times without losing the resolution of the axial method.

A second possible configuration of a method for spectrum detection is presented in Fig. 22. Due to the fact that the change of the distance between the components of the optical frequency comb (if they are registered in domain λ) simultaneously with moderate wavelength increase, it is possible to design the spectrometer in such a way that the individual components of the optical frequency comb fall exactly into the individual pixels on the matrix of photosensitive elements or individual photosensitive elements of the multi-element detection device. In such a configuration step 445 is not required, this being the step in which the positions of individual components in the matrix of photosensitive elements are set, because they are known if the spectrometer has been properly designed. This information is used in step 455 of the algorithm presented in Fig. 17, in which an appropriate choice of points of a registered spectrum is made so as to create a spectrum in domain k. This method of spectrum detection eliminates the need for interpolation of spectrum values and at the same time allows a further increase in the speed of numerical processing of registered data.

Fig. 16 presents another variant of spectrum detection, shown in Fig. 13 as step 430, which starts from creating the resultant spectrum from the object light beam and the reference light beam in step 426. In step 427 the resultant spectrum is formed in such a way that the components of the optical frequency comb fall upon every second photosensitive element of the matrix of photosensitive elements or every second photosensitive element of the multi-element detection device. Next, in step 428, the resultant spectrum is registered by using a CCD device option for reading every second pixel or photosensitive element, and electrical signals generated by the photosensitive elements of the multi-element detection device are transmitted to the calculation unit in step 429. This variant of spectrum detection has all the advantages of the two earlier configurations, and also significantly reduces the decrease of spectrometer resolution caused by charge leakage in the matrix of photosensitive elements in the form of CCD devices. In this configuration the spectrometer is designed in such a way that the individual components of optical frequency comb 211 fall into every second photosensitive element of the matrix 174 of photosensitive elements, which is shown in detail in Fig. 23. Thanks to such a solution any charge-leakage from lighted photosensitive elements moves to neighbouring photosensitive elements which hold no usable signals, and thus there is no decrease in the detector's spectral resolution and no decreasing signal in the form of the amplitude of the interference fringes modulating the optical frequency comb. Reading the spectrum in this configuration can be done in two ways: either by reading all photosensitive elements and selecting every second point from the collected data, or by using an option available in industrial CCD devices for reading only every *q-th* pixel or every *q-th* photosensitive element at each the component is registered, for example odd or even uneven pixels or photosensitive elements. This way doubles the reading-rate of spectra and simultaneously a spectrum directly positioned in domain k is created.

Alignment of the distances between the components of the component set and their correlation with the distances between the pixels or photosensitive elements in the multi-element detection device may be achieved in many ways. One way is to arrange the inter-mirror distances of the Fabry-Perot interferometer or the adjustable or tuned Fabry-Perot interferometer in such a way that every component of the resultant spectrum, this being the set or group of component modulated by the interference signal falls into every q-th pixel or photosensitive element of the multi-element detection device that means these distances must correspond with the photosensitive elements of the multi-element detection device, in which case q must be greater than 0.

In the second method angles between the combined light beam and the device separating spatially wavelength components are set to specific value so that the components of the resultant beam, forming the set or group of components modified by the interference signal such that again, they correspond to the photosensitive elements of the multi-element detection device and every component of the resultant spectrum reaches every q-th pixel or photosensitive element of the multi-element detection device, in which case q must be greater than 0.

Another method concerns the case where the angle of the combined light beam directed towards the multi-element detection device in such a way that the angle of incidence of the combined light beam on the device separating spatially wavelength components is adjusted such that the individual components of the resultant spectrum of the combined light beam are matched to every *q-th* photosensitive element of the multi-element detection device, where q > 0.

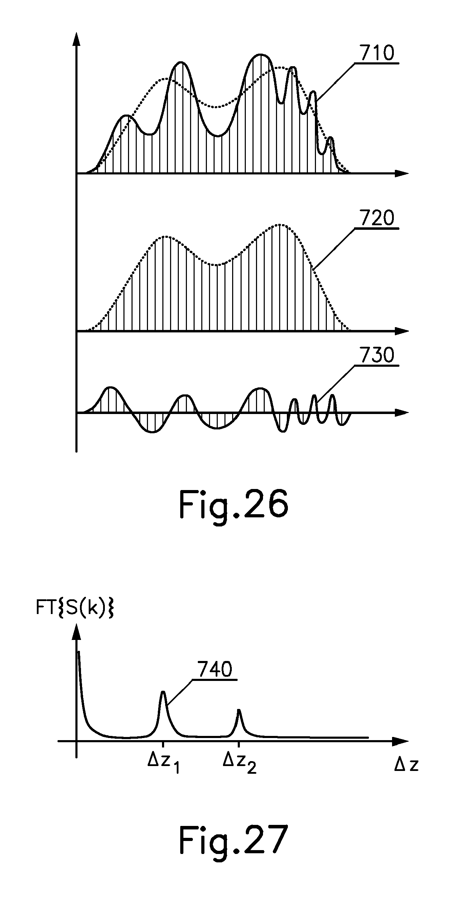

Fig. 17 presents one variant of data analysis, which begins in step 441 with zero setting of i and j. In step 442 a calculation unit reads positions of optical frequency comb components from a resultant compound spectrum assembled in a multiplex way in step 460. The result of this operation is a vector of component positions. In step 443 it is checked if j is higher than M. If j is lower than M, then in step 444, i is set to zero and in step 447 another set of N spectra is selected from the registered M sets made available in step 445. In step 450, N spectra of a given set are combined into one spectrum consisting of spectra made available in step 451. In step 452, the background spectrum is subtracted from the combined multiple spectrum, as described in Fig. 26. In step 455 the resultant multi spectrum is decoded such that only those points are chosen from the resultant multi spectrum which correspond to values from the vector position of components selected in step 445 and made available in step 453. The decoded resultant multi spectrum is then received directly in wavenumber domain k. In step 470 the decoded resultant multi spectrum is analysed in terms of frequency, for instance, by applying the Fourier transformation, producing a single line - called an A-scan - of a cross-sectional image of examined object, which is recorded in the calculation unit in step 471. If it is not the last A-scan, then in step 475 j is increased by 1. Analysis of data can be made after completing the second loop of recording and collecting data in steps 460 or 560 of the algorithms shown in Figs. 13 and 18, but the process may occur both inside the first or the second loop.

Fig. 18 shows another algorithm for imaging objects. The procedure begins by generating the light beam and forming the optical frequency comb in step 505 and dividing the light beam into a reference light beam and an object light beam in step 506 and establishing what will be referred to as a background spectrum by directing the object beam outside the object in step 511. In step 512 recording is made of the background spectrum. In step 513 the object light beam is set at the beginning of the scanning path whilst in step 514 $i$ and $j$ are set to zero. In step 515 it is checked if $i$ is higher than N. If i is equal to or lower than N, changes of positions of the optical frequency comb components take place in step 516, after which in step 520 interference of object and reference beams occurs, as described in steps 415 - 418. In step 530 detection of the resultant spectrum of the combined light beam is made and it is transferred into an electrical signal, which is performed as described in reference to step 430. Next, i is increased by 1 and the procedure is continued in step 515. When the light beam reaches its final position of the scanning path, then in step 535 it is checked whether j is equal to M, which would signify that the whole object has been scanned. If j is lower than M, the procedure is continued in step 536, in which a change of the position of the object light beam takes place, after which i is reset to zero, and j is increased by 1 in step 537 and the procedure is moved to step 515. After scanning the entire object, data analysis is made in step 560, and in step 580 all calculated A-scans are displayed side by side, and the final result is a cross-section or a three dimensional representation of the examined object, depending whether scanning by the object beam was made in one or in two axes perpendicular to the trajectory of the object beam.

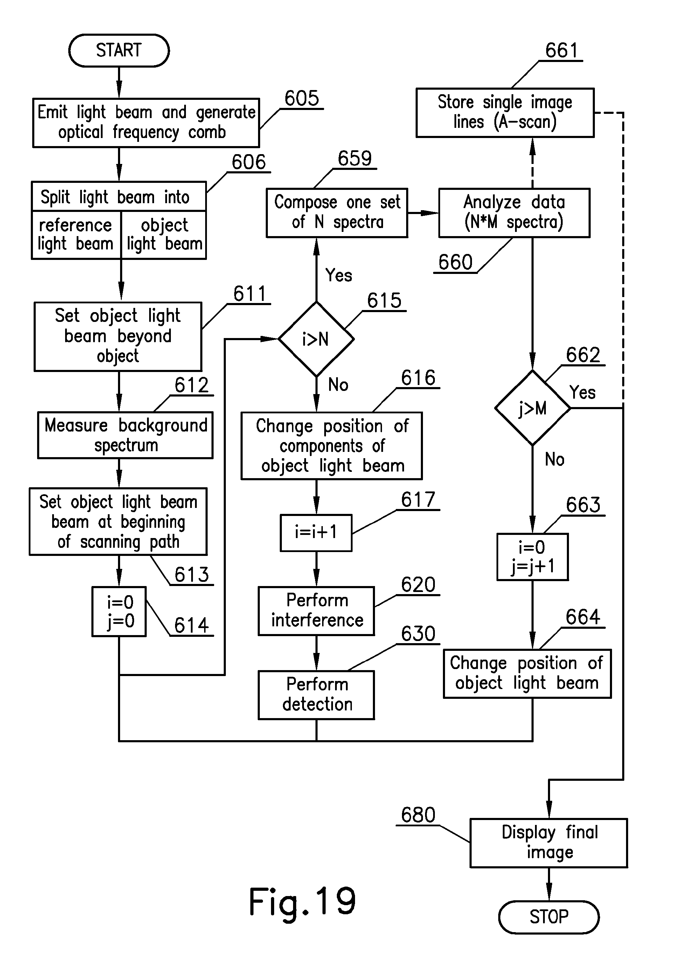

Fig. 19 shows yet another algorithm for imaging objects. As in previous variants of object imaging algorithms, a procedure begins from the generation of a light beam and forming of the optical frequency comb, which is carried out in step 605, beamsplitting into reference and object beams in step 606 and setting the object light beam beyond the object in step 611. In step 612 background measurement is made, and in step 613 the object light beam is set at the beginning of its scanning path. In step 614 i and j are reset to zero. In step 615 it is checked if $i$ is higher than N. If $i$ is lower than N, a change of the position of the optical frequency comb components takes place in step 616, after which, in step 620, interference of the object and reference beams occurs, as described in steps 415 - 418. In step 630 the spectrum is detected.

If it turns out after checking in step 615, that i is higher, in step 659 one set of N spectra is registered, and in step 660 data analysis is made regarding N spectra, which includes subtracting background spectrum from every one of registered N spectra, decoding spectra by using vector of components' positions receiving partial spectra, assembly of partial spectra into a compound spectrum, making frequency analysis and as a result of which one line is received, so called one A-scan. Decoding the spectrum involves the use of the vectors of component positions to yield sub-spectra, combining these into a resultant spectrum, performing frequency analysis to obtain a one-line A-scan which provides information about a discrete linear part of the image aligned with the object light beam and which has its width and a length equal to that of the object being examined. If the object light beam has a circular cross-section the part of the object being scanned forms a cylinder and when the beam is formed with a linear cross-section the obtained image corresponds to a cubicoidal fragment of the examined object. In step 661 single lines of an image are collected. In step 662 it is checked whether j is higher than M, and in case when j is lower than M, then in step 663 i is reset to zero, and j is increased by 1 and the procedure is continued in step 615, repeating mentioned steps M times, as a result getting a ready image consisting of M lines, which is displayed in step 680.

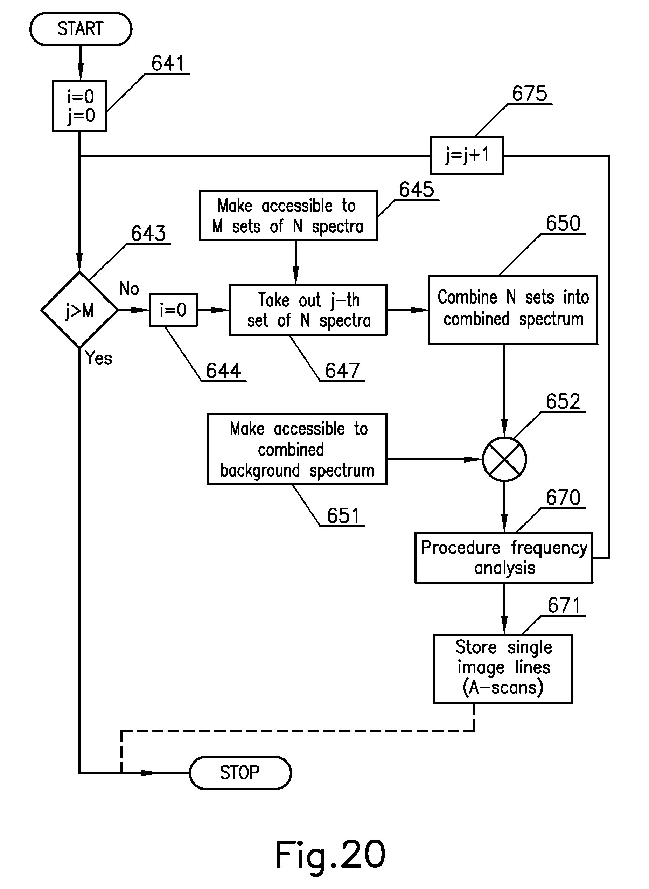

Fig. 20 presents another variant of data analysis, which is used in the algorithm shown in Fig. 19, and which begins in step 641 by setting to zero values i and j. In step 643 it is checked if j is higher than M. If j is lower than M, in step 644 $i$ is reset to zero, and in step 647 another set of N spectra is chosen out of the registered M sets made accessible in step 645. In step 650, N spectra of a given set are assembled into one compound spectrum. In step 652 from compound spectrum, a compound spectrum of background made accessible in step 651 is subtracted, according to the way presented in Fig. 26, in which from the resultant spectrum 710 the background spectrum 720 is subtracted and a resultant compound spectrum 730 is received. In step 670 frequency analysis of resultant compound spectrum 730 is made, for example by calculating Fourier transformation, receiving an object single line 740 of cross-section image of examined object called A-scan, shown in Fig. 27. Peaks at distances $\Delta z_1$ and $\Delta z_2$ from a datum surface correspond to two back-scattering or reflecting points creating an axial structure of reconstructed object. The single object line is registered in step 671. If it is not the last A-scan, then in step 675 j is increased by 1.

In comparison with hitherto known methods of OCT imaging, the presented solution has numerous advantages. Thanks to this solution there is a significant reduction of limitations as known in the SOCT method, connected with averaging of the interference signal after sub-dividing the frequency band corresponding to photosensitive element width due to the fact, that single photosensitive elements register monochromatic radiation fairly accurately. Moreover, this solution eliminates the need for numerical calculation of the registered spectrum from domain of wave-lengths to optical frequencies due to the fact that components of quasi-discrete spectrum registered by spectrometer are equally spaced in the domain of optical frequencies. Thanks to this solution, the time of data processing has been reduced due to the significantly shorter operational time to interpolate the values of the recorded spectra in the positions set by the components of the optical frequency comb as compared with the numerical calculation of the spectra applied in currently known SOCT techniques. In case of a configuration in which the components fall into exactly positioned pixels of the matrix of photosensitive elements, the spectra are registered directly in the domain of the wavenumber k, and no additional numerical operations are required, which further shortens numerical data processing. The significant advantage of such a solution is an increase in spectrometer resolution by minimising the effect of charge leakage in the CCD detectors by such adjustment of distances between the components of the optical frequency comb so as to make the components fall exactly into a $q$-th cell or photosensitive element of the matrix of photosensitive elements, in a specific case onto every second photosensitive element. The effect of charge leakage in this method of detection still exists, but it does not influence the usable signal, because leaking charges from a given pixel move to neighbouring pixels, which are not used to construct the spectrum being recorded. It is true that in this way half of the photosensitive elements of the detector are unavailable (in the variant in which every second photosensitive element is illuminated), but the drawback can be easily averted by using a multi-element detection device with twice the density of photosensitive cells or elements. Furthermore, the limitations of the SSOCT system connected with mode hopping are eliminated when a light source and the Fabry-Perot interferometer of wide spectrum (low time coherence) is used to generate the optical frequency comb. This results in an effective increase of the imaging range achieved by increasing the effective number of interference fringes sampling signals in a process of multiple spectrum registration and assembling them into a spectrum consisting of higher number of points.

The presented apparatus and the method for imaging the interior structure of objects allow their complex measurement [13], in which two or more spectrum measurements at each position of the object light beam may be performed. The spectra are measured in that way, the phase of the interference fringes between neighbouring spectra form a fraction of the wave length. In contemporary systems, reference mirrors mounted on the positioning device 131, as a shown in Fig 8A are used, which give only a small delay in the reference light beam relative to the object light beam that takes place in the shifting of the interference fringes. In the proposed variant of the apparatus for complex measurement, instead of the phase-change, thanks to the movement of the reference mirror, the phase changes are achieved by changes in the position of the components of the optical frequency comb in the domain of k The creation of images using this method has been described in several publications [13] and is realised using mathematical methods, by which information regarding the phase and amplitude of the interference fringes may be obtained.

## Claims

1. An apparatus for optical frequency domain tomography comprising a generating device (110) emitting a light beam with a defined spectrum, a means (120) for splitting the light beam into at least one object light beam and at least one reference light beam, a device (120) forming a combined light beam from at least one reference light beam and at least one object light beam, and a device (170) for spectral analysis of the combined light beam, **characterised in that** the generating device (111) emitting the light beam is either also a device (110) transforming the light beam with the defined spectrum into a set of components or an optical frequency comb having components (211) spaced at discrete, defined distances, or in a path of the light beam emitted by the generating device (111) or in the paths of at least one reference light beam (208) and at least one object light beam (209) is placed a device (113) transforming the light beam or the reference light and object light beams (208, 209) into a set (212) of components or an optical frequency comb or optical frequency combs, as applicable, having components (211) spaced at discrete, defined distances, whereas the means (120, 320) for splitting the light beam into at least one object light beam (230) and at least one reference light beam (220) and the device (120, 340) forming the combined light beam from at least one reference light beam (220, 221) and at least one object light beam (230, 231) are capable to process the optical frequency combs, and the device (170) for spectral analysis of the combined light beam is provided with a multi-element detection device (174) having photosensitive elements (173) capable of registering more than one component (211) of the optical frequency comb, and each of the sensitive elements (173) of the multi-element detection device (174) registers at most one component (211) of the optical frequency comb, and wherein the photosensitive elements (173) of the multi-element detection device (174) are spaced at distances correlating with the discrete defined distances between the components (211) of the optical frequency comb.

2. The apparatus for optical frequency domain tomography according to claim 1 **characterised in that** the device (113) transforming the light beam with the defined spectrum into the optical frequency comb or the set of the optical components is a Fabry-Perot interferometer or an adjustable or tuned scanning Fabry-Perot interferometer or an etalon or an arrangement basing on modulation of a phase or amplitude of coherence laser light.

3. The apparatus for optical frequency domain tomography according to claim 1 **characterised in that** the Fabry-Perot interferometer (113) or the adjustable or tuned scanning Fabry-Perot interferometer or the etalon is fitted with a positioning device (114) for changing a distance between reflective mirror surfaces.

4. The apparatus for optical frequency domain tomography according to claim 1 **characterised in that** the means for splitting the light beam is either at least one cube or rectangular prism beamsplitter or at least one fiber-coupler (320) or at least one semi-transparent mirror.

5. The apparatus for optical frequency domain tomography according to claim 1 **characterised in that** in the path of the object light beam (230) is placed at least one element (150) focusing the object light beam.

6. The apparatus for optical frequency domain tomography according to claim 1 **characterised in that** in the path of the object light beam (230) and/or the reference light beam (220) is placed at least one optical arrangement (150) modifying a cross-section shape of the object light beam and/or the reference light beam.

7. The apparatus for optical frequency domain tomography according to claim 1 **characterised in that** in the path of the object light beam (230) is placed an arrangement (140) adjusting a position of the object light beam relative to the examined object.

8. The apparatus for optical frequency domain tomography according to claim 1 **characterised in that** an object (160) is placed on an apparatus (161) adjusting a position of the object (160) relative to the object light beam (230).

9. The apparatus for optical frequency domain tomography according to claim 1 **characterised in that** in the path of the reference light beam (220) is placed a reference mirror (130) having an adjustable position.

10. The apparatus for optical frequency domain tomography according to claim 1 **characterised in that** device (340) forming the combined light beam from at least one reference light beam and at least one object light beam is either at least one cube or rectangular prism beamsplitter or at least one fiber-coupler or at least one semi-transparent mirror.

11. The apparatus for optical frequency domain tomography according to claim 1 **characterised in that** in the path of the reference and/or the object light beam is placed an arrangement (330) to equal the pathlengths of the object light beam and of the reference light beam.

12. The apparatus for optical frequency domain tomography according to claim 1 **characterised in that** the device (170) for spectral analysis of the combined light beam is fitted with a means (171) splitting the combined light beam into a resultant spectrum.

13. The apparatus for optical frequency domain tomography according to claim 1 **characterised in that** the multi-element detection device (174) is a matrix or a line of photosensitive elements.

14. The apparatus for optical frequency domain tomography according to claim 1 **characterised in that** the device for spectral analysis of the combined light beam is provided with a calculation unit (190) for transforming the resultant spectrum into an image of the object.

15. The apparatus for optical frequency domain tomography according to claim 1 **characterised in that** the device (113) transforming the light beam with a defined spectrum into the optical frequency comb or the set of the optical components having the components spaced at discrete, defined distances is fitted with a positioning device (114) adjusting the distances between the optical components of the set of the optical components to the distances between the photosensitive elements of the multi-element detection device.

16. The apparatus for optical frequency domain tomography according to claim 1 **characterised in that** the device (170) for spectral analysis of the combined light beam is fitted with an arrangement (172) for adjusting the distances between the optical components of the set of the optical components to the distances between the photosensitive elements of the multi-element detection device.

17. A method for imaging of objects by applying optical frequency domain tomography using an optical frequency comb **characterised in that** the method comprises the following steps:

   generating a light beam having a spectrum comprising a set of optical components and/or forming an optical frequency comb;
   splitting the set of optical components into at least one reference set of optical components and at least one object set of optical components;

directing onto an object at least one object set of optical components and forming a returning object set of optical components;

transforming at least one reference set of optical components and at least one returning object set of optical components into a combined light beam;

forming a resultant spectrum of the combined light beam having optical components spaced at discrete, defined distances;

registering the resultant spectrum of the combined light beam by a detection device fitted with photosensitive elements spaced at distances correlated with the discrete defined distances of the optical components of the combined light beam and

transforming the resultant spectrum of the combined light beam into a data stream enabling the visualization of the examined object.

18. The method for imaging of objects according to claim 17 **characterised in that** generating the light beam in the form of the optical frequency comb or the set of the optical components is conducted by a Fabry-Perot interferometer or an adjustable or tuned scanning Fabry-Perot interferometer placed in a generating device emitting the light beam or after the generating device emitting the light beam.

19. The method for imaging of objects according to claim 17 **characterised in that** generating the light beam as the optical frequency comb or the set of the optical components is conducted by a phase or amplitude modulator placed in a generating device emitting the light beam or after the generating device emitting the light beam.

20. The method for imaging of objects according to claim 17 **characterised in that** generating the light beam as the optical frequency comb or the set of the optical components is characteristic type of light emission for used light source.

21. The method for imaging of objects according to claim 17 **characterised in that** forming the spectrum of the combined light beam is performed by a spectrometer.

22. The method for imaging of objects according to claim 17 **characterised in that** registering the spectrum of the combined light beam is performed by a multi-element detection device fitted with photosensitive elements.

23. The method for imaging of objects according to claim 17 **characterised in that** arranging a distance between reflective planes of the Fabry-Perot interferometer or the adjustable or tuned Fabry-Perot interferometer to illuminate every $q$-$th$ photosensitive element of the multi-element detection device, where $q > 0$, with successive components of the resultant spectrum, this being the set or group of component modulated by the interference signal.

24. The method for imaging of objects according to claim 17 **characterised in that** an angle between the combined light beam and the device separating spatially wavelength components is chosen to illuminate every q-th photosensitive element of the multi-element detection device, where q > 0, with successive components of the resultant spectrum, this being the set or group of component modulated by the interference signal.

25. The method for imaging of objects according to claim 17 **characterised in that** an angle of incidence of the resultant spectrum onto the multi-element detection device is chosen to project successive components of the resultant spectrum, this being the set or group of component modulated by the interference signal, into every q-th photosensitive element of the multi-element detection device, where q > 0.

26. The method for imaging of objects according to claim 17 **characterised in that** only signals from every q-th photosensitive element of the multi-element detection device from signals generated by the components of the resultant spectrum projected onto the multi-element detection device are chosen.

27. The method for imaging of objects according to one of claims 23, 24, 25, 26 **characterised in that** $q = 2$.

28. The method for imaging of objects according to claim 17 **characterised in that** transforming spectra to an image of objects is performed by calculating Fourier transformations and imaging all Fourier transformations as a light intensity image.

Fig.1

Fig.2

EP 1 870 028 A1

Fig.3

18

Fig.4

Fig.5

Fig.6

Fig.7

Fig.8A

Fig.8B

Fig.8C

Fig.9

Fig.10

Fig.11

Fig.12

```
                    ┌─────────────┐
                    │    START    │
                    └──────┬──────┘
                           │
              ┌────────────▼────────────┐
              │ Emit light beam and     │  405
              │ generate optical        │
              │ frequency comb          │
              └────────────┬────────────┘
                           │
              ┌────────────▼────────────┐  406
              │ Split light beam into   │
              ├────────────┬────────────┤
              │ reference  │ object     │
              │ light beam │ light beam │
              └────────────┴────────────┘
```

Fig.13

START

405 — Emit light beam and generate optical frequency comb

406 — Split light beam into reference light beam | object light beam

459 — Store M sets of N spectra

439 — Set object light beam beyond the object

409 — i>N  First loop  Second loop  Yes

435 — j>M

No — 420 — Perform interference

436 — i=0

430 — Perform detection

437 — Change position of object light beam

431 — Change position of components of optical frequency comb

438 — j=j+1

440 — Measure background spectrum

432 — i=i+1

407 — Set object light beam at beginning of scanning path

408 — i=0 j=0

460 — Analyse data (N*M spectra)

480 — Display final image

STOP

START

415

Delay reference
light beam

416

Perform correction
of dispersion

417

Direct light beam to
object and perform light
scattering in structure of object

Combine reference
light beam
and object light beam

418

STOP

Fig.14

START

Split combined light beam into spectrum — 421

Image spectrum on matrix of photodetectors — 422

Register spectrum — 423

Send spectrum to computer — 424

STOP

Fig.15

START

Split combined light beam into spectrum — 426

Image spectrum on matrix of photodetectors in so way that components of optical frequency comb are directed to every second photodetector — 427

Register spectrum applying CCD camera having option to read every second photodetector — 428

Send spectrum to computer — 429

STOP

Fig.16

START

441
i=0
j=0

442
Read position of components
of optical frequency comb

475
j=j+1

445
Make accessible to M
sets of N spectra

443
j>M

No

i=0
444

Take out j-th set
of N spectrum
447

450
Combine N set into
combined spectrum

452

451
Make accessible to
combined background
spectrum

453
Make accessible to vector of
positions of components of
optical frequency comb

455
Interpret resultant
spectrum

Yes

470
Procedure frequency
analysis

471
Store single
image lines
(A-scans)

STOP

Fig.17

START

Emit light beam and
generate optical frequency — 505

Split light beam into — 506

| reference light beam | object light beam |

Set object light
beam beyond object — 511

Measure
background spectrum — 512

Set object light
beam at beginning of
scanning path — 513

i=0
j=0 — 514

i>N — 515

Yes

No

Change position of
components of optical
frequency comb — 516

Perform
interference — 520

Perform
detection — 530

i=i+1 — 531

j>M — 535

Yes

No

Change position of
object light beam — 536

i=0
j=j+1 — 537

Analyze data
(N*M spectra) — 560

Display final
image — 580

STOP

Fig.18

START

Emit light beam and generate optical frequency comb — 605

Split light beam into — 606
| reference light beam | object light beam |

Set object light beam beyond object — 611

Measure background spectrum — 612

Set object light beam beam at beginning of scanning path — 613

i=0 j=0 — 614

i>N — 615

Yes → Compose one set of N spectra — 659

No ↓

Change position of components of object light beam — 616

i=i+1 — 617

Perform interference — 620

Perform detection — 630

Analyze data (N*M spectra) — 660

Store single image lines (A-scan) — 661

j>M — 662

Yes

No ↓

i=0 j=j+1 — 663

Change position of object light beam — 664

Display final image — 680

STOP

Fig.19

29

START

641
i=0
j=0

675
j=j+1

645
Make accessible to
M sets of N spectra

650
Combine N sets into
combined spectrum

643
j>M   No   644   i=0   647   Take out j-th
set of N spectra

Yes

652

651
Make accessible to
combined
background spectrum

670
Procedure frequency
analysis

671
Store single
image lines
(A-scans)

STOP

Fig.20

Fig.21

Fig.22

Fig.23

Fig.24

Fig.25

710

720

730

Fig.26

FT{S(k)}

740

$\Delta z_1$    $\Delta z_2$    $\Delta z$

Fig.27

**European Patent Office**

## EUROPEAN SEARCH REPORT

Application Number

EP 06 46 1008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2007/003288 A (CARL ZEISS MEDITEC AG) 11 January 2007 (2007-01-11) * page 9, last paragraph - page 11, last paragraph * * page 13, paragraph 3 * * page 17, paragraph 3 * * page 22, paragraph 3 - page 24, paragraph 1 * * figures 3,4 * ----- | 1-14, 17-26,28 | INV. A61B5/00 G01N21/47 G01B9/02 |
| X  A | US 2005/018201 A1 (DE BOER JOHANNES F [US] ET AL) 27 January 2005 (2005-01-27)  * paragraph [0080] * * paragraphs [0091] - [0098] * * paragraphs [0105] - [0107] * * paragraphs [0135] - [0138] * * figures 2,5 * ----- | 17, 20-22, 24-26,28  1,4-14 | |
| A | WO 2006/019181 A (AGRICULTURE AND TECHNOLOGY NAT [JP]; KUROKAWA TAKASHI [JP]; TANAKA YOU) 23 February 2006 (2006-02-23) * abstract * * figures 1,6 * ----- | 1,17 | TECHNICAL FIELDS SEARCHED (IPC) A61B G01N G01B |
| A | CHOI S ET AL: "Frequency-comb-based interferometer for profilometry and tomography" Optics Letters Opt. Soc. America USA, vol. 31, no. 13, 13 April 2006 (2006-04-13), pages 1976-1978, XP002411649 ISSN: 0146-9592 * the whole document * ----- | 1,17 | |

The present search report has been drawn up for all claims

| Place of search | Date of completion of the search | Examiner |
|---|---|---|
| Berlin | 11 January 2007 | Völlinger, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 46 1008

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | Publication date | Patent family member(s) | Publication date |
|---|---|---|---|
| WO 2007003288 A | | NONE | |
| US 2005018201 A1 | 27-01-2005 | NONE | |
| WO 2006019181 A | 23-02-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004043245 A1
- WO 2006017837 A2
- WO 2006036717 A2
- US 20010045513 A1
- US 20026493091 B2
- US 20050036150 A1
- GB 2416451 A

### Non-patent literature cited in the description

- **D. HUANG ; E. A. SWANSON ; C. P. LIN ; J. S. SCHUMAN ; W. G. STINSON ; W. CHANG ; M. R. HEE ; T. FLOTTE ; K. GREGORY ; C. A. PULIAFITO.** Optical Coherence Tomography. *Science,* 1991, vol. 254, 1178-1181
- **E. A. SWANSON ; J. A. IZATT ; M. R. HEE ; D. HUANG ; C. P. LIN ; J. S. SCHUMAN ; C. A. PULIAFITO ; J. G. FUJIMOTO.** In vivo retinal imaging by optical coherence tomography. *Opt. Lett.,* 1993, vol. 18 (21), 1864-1866
- **C. A. PULIAFITO ; M. R. HEE ; C. P. LIN ; E. REICHEL ; J. S. SCHUMAN ; J. S. DUKER ; J. A. IZATT ; E. A. SWANSON ; J. G. FUJIMOTO.** Imaging of macular diseases by optical coherence tomography. *Ophthalmol.,* 1995, vol. 102 (2), 217-229
- **A. F. FERCHER ; C. K. HITZENBERGER ; G.KAMP ; S.Y.EI-ZAIAT.** Measurements of intraocular distances by backscattering spectral interferometry. *Opt. Commun.,* 1995, vol. 117, 43-48
- **G. HAUSLER ; M. W. LINDNER.** oherence Radar and Spectral Radar - new tools for dermatological diagnosis. *J. Biomed. Opt.,* 1998, vol. 3 (1), 21-31
- **R. A. LEITGEB ; C. K. HITZENBERGER ; A. F. FERCHER.** Performance of Fourier domain vs. time domain optical coherence tomography. *Optics Express,* 2003, vol. 11, 889-894
- **J. F. DE BOER ; B. CENSE ; B. H. PARK ; M. C. PIERCE ; G. J. TEARNEY ; B. E. BOUMA.** Improved signal-to-noise ratio in spectral-domain compared with time-domain optical coherence tomography. *Opt. Lett.,* 2003, vol. 28, 2067-2069
- **M. A. CHOMA ; M. V. SARUNIC ; C. YANG ; J. A. IZATT.** Sensitivity advantage of swept source and Fourier domain optical coherence tomography. *Optics Express,* 2003, vol. 11, 2183
- **N. NASSIF ; B. CENSE ; B. H. PARK ; S. H. YUN ; T. C. CHEN ; B. E. BOUMA ; G. J. TEARNEY ; J. F. DE BOER.** In vivo human retinal imaging by ultrahigh-speed spectral domain optical coherence tomography. *Opt. Lett.,* 2004, vol. 29, 480-482
- **S. H. YUN ; G. J. TEARNEY ; B. E. BOUMA ; B. H. PARK ; J. F. DE BOER.** High-speed spectral-domain optical coherence tomography at 1.3 $\mu$m wavelength. *Optics Express,* 2003, vol. 11, 3598-3604
- **N. A. NASSIF ; B. CENSE ; B. H. PARK ; M. C. PIERCE ; S. H. YUN ; B. E. BOUMA ; G. J. TEARNEY ; T. C. CHEN ; J. F. DE BOER.** In vivo high-resolution video-rate spectral-domain optical coherence tomography of the human retina and optic nerve. *Optics Express,* 2004, vol. 12, 367-376
- **R. LEITGEB ; L. SCHMETTERER ; F. BERISHA ; C. K. HITZENBERGER ; M. WOJTKOWSKI ; T. BAJRASZEWSKI ; A. F. FERCHER.** Real- time measurements of in-vitro flow by Fourier domain optical coherence tomography. *Opt. Lett.,* 2004, vol. 29, 171-174
- **M.WOJTKOWSKI ; A. KOWALCZYK ; R. LEITGEB ; A. F.FERCHER.** Full range complex spectral optical coherence tomography. *Opt. Lett.,* 2002, vol. 27, 1415-1418
- **R. A. LEITGEB ; C.K. HITZENBERGER ; T. BAJRASZEWSKI ; A. F. FERCHER.** Phase shifting method to achieve high speed long depth range imaging using FDOCT. *Opt. Lett.,* 2003, vol. 28, 2201-2204
- **A.H. BACHMANN ; R.A. LEITGEB ; T. LASSER.** Heterodyne Fourier domain optical coherence tomography for full range probing with high axial resolution. *Optics Express,* 2006, vol. 14 (4), 1487-1496
- **P. TARGOWSKI ; M. SZKULMOWSKI ; M.WOJTKOWSKI ; A. KOWALCZYK.** Improved complex spectral domain OCT for in vivo eye imaging. *Opt. Commun.,* 2005, vol. 249, 357-362

- **S. R. CHINN ; E. A. SWANSON ; J. G. FUJIMOTO.** Optical coherence tomography using a frequency-tunable optical source. *Opt. Lett.,* 1997, vol. 22 (5), 340-342

- **M. A. CHOMA ; K. HSU ; J. A. IZATT.** Swept source optical coherence tomography using an all-fiber 1300-nm ring laser source. *J. Biomed. Opt.,* 2005, vol. 10 (4

- **A. NUSSBAUM ; R. A. PHILLIPS.** Contemporary optics for scientists and engineers. Prentice-Hall, Inc, 1976, 165-174